# EUROPEAN PATENT SPECIFICATION

(11) **EP 1 660 649 B1**
(45) Date of publication and mention of the grant of the patent: **12.12.2007**
(21) Application number: 04786904.5
(22) Date of filing: 01.09.2004
(51) Int. Cl.: C12N 9/10, C12N 1/19, C12N 15/82, C12N 15/54, A01H 5/00

(54) **METHOD FOR DETOXIFICATION OF MYCOTOXINS**
VERFAHREN ZUR ENTGIFTUNG VON MYKOTOXINEN
METHODE DE DETOXICATION DE MYCOTOXINES

(30) Priority: 01.09.2003 EP 03450194
(43) Date of publication of application: 31.05.2006
(73) Proprietor: Universität für Bodenkultur Wien, 1180 Wien (AT)
(72) Inventor: POPPENBERGER, Brigitte, York YO10 4BQ (GB); ADAM, Gerhard, A-1160 Vienna (AT); BERTHILLER, Franz, A-1050 Vienna (AT); KRSKA, Rudolf, A-3430 Tulln (AT); KUCHLER, Karl, A-1180 Vienna (AT); LUSCHNIG, Christian, A-1170 Vienna (AT); GLÖSSL, Josef, A-1140 Vienna (AT); LUCYSHYN, Doris, A-1030 Vienna (AT); SCHUHMACHER, Rainer, A-3430 Tulln (AT); SIEBERER, Tobias, York YO10 4BQ (GB)
(74) Representative: Sonn & Partner Patentanwälte
(86) International application number: PCT/EP2004/009728
(87) International publication number: WO 2005/021740

(56) References cited:
- WO-A-00/00626
- WO-A-97/45546
- WO-A-98/34471
- VON RAD UTA ET AL: "Two glucosyltransferases are involved in detoxification of benzoxazinoids in maize" PLANT JOURNAL, vol. 28, no. 6, December 2001 (2001-12), pages 633-642, XP002276049 ISSN: 0960-7412
- YAMAZAKI MAMI ET AL: "Molecular cloning and biochemical characterization of a novel anthocyanin 5-O-glucosyltransferase by mRNA differential display for plant forms regarding anthocyanin" JOURNAL OF BIOLOGICAL CHEMISTRY, vol. 274, no. 11, 12 March 1999 (1999-03-12), pages 7405-7411, XP002276050 ISSN: 0021-9258
- LIM ENG-KIAT ET AL: "Regioselectivity of glucosylation of caffeic acid by a UDP-glucose: Glucosyltransferase is maintained in planta." BIOCHEMICAL JOURNAL, vol. 373, no. 3, 1 August 2003 (2003-08-01), pages 987-992, XP002276051 ISSN: 0264-6021
- JACKSON ROSAMOND G ET AL: "Over-expression of an Arabidopsis gene encoding a glucosyltransferase of indole-3-acetic acid: Phenotypic characterisation of transgenic lines." PLANT JOURNAL, vol. 32, no. 4, November 2002 (2002-11), pages 573-583, XP002276052 ISSN: 0960-7412
- YAMAZAKI MAMI ET AL: "Two flavonoid glucosyltransferases from Petunia hybrida: Molecular cloning, biochemical properties and developmentally regulated expression" PLANT MOLECULAR BIOLOGY, vol. 48, no. 4, March 2002 (2002-03), pages 401-411, XP001180742 ISSN: 0167-4412
- OKUBARA P A ET AL: "Engineering deoxynivalenol metabolism in wheat through the expression of a fungal trichothecene acetyltransferase gene." THEORETICAL AND APPLIED GENETICS, vol. 106, no. 1, December 2002 (2002-12), pages 74-83, XP001180744 ISSN: 0040-5752 (ISSN print)
- SEWALD N ET AL: "Structure elucidation of a plant metabolite of 4-desoxynivalenol" TETRAHEDRON ASYMMETRY 1992 UNITED KINGDOM, vol. 3, no. 7, 1992, pages 953-960, XP002276053 ISSN: 0957-4166
- MESSNER BURKHARD ET AL: "Arabidopsis glucosyltransferases with activities toward both endogenous and xenobiotic substrates." PLANTA (BERLIN), vol. 217, no. 1, May 2003 (2003-05), pages 138-146, XP001180743 ISSN: 0032-0935
- MILLER J D ET AL: "DEGRADATION OF DEOXYNIVALENOL BY SUSPENSION CULTURES OF THE FUSARIUM HEAD BLIGHT RESISTANT WHEAT CULTIVAR FRONTANA" CANADIAN JOURNAL OF PLANT PATHOLOGY, vol. 8, no. 2, 1986, pages 147-150, XP009028930 ISSN: 0706-0661
- GORST-ALLMAN C P ET AL: "STRUCTURE ELUCIDATION OF A NOVEL TRICHOTHECENE GLYCOSIDE USING PROTON NMR AND CARBON-13 NMR SPECTROSCOPY" JOURNAL OF THE CHEMICAL SOCIETY PERKIN TRANSACTIONS I, no. 7, 1985, pages 1553-1556, XP009029031 ISSN: 0300-922X
- SCHAEFFNER A ET AL: "Genes and enzymes for in-planta phytoremediation of air, water and soil" ACTA BIOTECHNOLOGICA, vol. 22, no. 1-2, 2002, pages 141-152, XP009028932 ISSN: 0138-4988

### SEQUENCE LISTING

<110> Austria Wirtschaftsservice Gesellschaft mit beschränkter Haftung Adam, Gerhard
<120> Glucosyltransferase
<130> R 42056
<140> EP 03450194.0
   <141> 2003-09-01
<160> 39
<170> PatentIn version 3.1
<210> 1
   <211> 495
   <212> PRT
   <213> Arabidopsis thaliana
<400> 1
   Met Val Ser Glu Thr Thr Lys Ser Ser Pro Leu His Phe Val Leu Phe
   1               5                   10                  15
   Pro Phe Met Ala Gln Gly His Met Ile Pro Met Val Asp Ile Ala Arg
               20                  25                  30
   Leu Leu Ala Gln Arg Gly Val Ile Ile Thr Ile Val Thr Thr Pro His
           35                  40                  45
   Asn Ala Ala Arg Phe Lys Asn Val Leu Asn Arg Ala Ile Glu Ser Gly
       50                  55                  60
   Leu Pro Ile Asn Leu Val Gln Val Lys Phe Pro Tyr Leu Glu Ala Gly
   65                  70                  75                  80
   Leu Gln Glu Gly Gln Glu Asn Ile Asp Ser Leu Asp Thr Met Glu Arg
                   85                  90                  95
   Met Ile Pro Phe Phe Lys Ala Val Asn Phe Leu Glu Glu Pro Val Gln
               100                 105                 110
   Lys Leu Ile Glu Glu Met Asn Pro Arg Pro Ser Cys Leu Ile Ser Asp
           115                 120                 125
   Phe Cys Leu Pro Tyr Thr Ser Lys Ile Ala Lys Lys Phe Asn Ile Pro
   130 135 140
   Lys Ile Leu Phe His Gly Met Gly Cys Phe Cys Leu Leu Cys Met His
   145 150 155 160
   Val Leu Arg Lys Asn Arg Glu Ile Leu Asp Asn Leu Lys Ser Asp Lys
   165 170 175
   Glu Leu Phe Thr Val Pro Asp Phe Pro Asp Arg Val Glu Phe Thr Arg
   180 185 190
   Thr Gln Val Pro Val Glu Thr Tyr Val Pro Ala Gly Asp Trp Lys Asp
   195 200 205
   Ile Phe Asp Gly Met Val Glu Ala Asn Glu Thr Ser Tyr Gly Val Ile
   210 215 220
   Val Asn Ser Phe Gln Glu Leu Glu Pro Ala Tyr Ala Lys Asp Tyr Lys
   225 230 235 240
   Glu Val Arg Ser Gly Lys Ala Trp Thr Ile Gly Pro Val Ser Leu Cys
   245 250 255
   Asn Lys Val Gly Ala Asp Lys Ala Glu Arg Gly Asn Lys Ser Asp Ile
   260 265 270
   Asp Gln Asp Glu Cys Leu Lys Trp Leu Asp Ser Lys Lys His Gly Ser
   275 280 285
   Val Leu Tyr Val Cys Leu Gly Ser Ile Cys Asn Leu Pro Leu Ser Gln
   290 295 300
   Leu Lys Glu Leu Gly Leu Gly Leu Glu Glu Ser Gln Arg Pro Phe Ile
   305 310 315 320
   Trp Val Ile Arg Gly Trp Glu Lys Tyr Lys Glu Leu Val Glu Trp Phe
   325 330 335
   Ser Glu Ser Gly Phe Glu Asp Arg Ile Gln Asp Arg Gly Leu Leu Ile
   340 345 350
   Lys Gly Trp Ser Pro Gln Met Leu Ile Leu Ser His Pro Ser Val Gly
   355 360 365
   Gly Phe Leu Thr His Cys Gly Trp Asn Ser Thr Leu Glu Gly Ile Thr
   370 375 380
   Ala Gly Leu Pro Leu Leu Thr Trp Pro Leu Phe Ala Asp Gln Phe Cys
   385 390 395 400
   Asn Glu Lys Leu Val Val Glu Val Leu Lys Ala Gly Val Arg Ser Gly
   405 410 415
   Val Glu Gln Pro Met Lys Trp Gly Glu Glu Glu Lys Ile Gly Val Leu
   420 425 430
   Val Asp Lys Glu Gly Val Lys Lys Ala Val Glu Glu Leu Met Gly Glu
   435 440 445
   Ser Asp Asp Ala Lys Glu Arg Arg Arg Arg Ala Lys Glu Leu Gly Asp
   450 455 460
   Ser Ala His Lys Ala Val Glu Glu Gly Gly Ser Ser His Ser Asn Ile
   465 470 475 480
   Ser Phe Leu Leu Gln Asp Ile Met Glu Leu Ala Glu Pro Asn Asn
   485 490 495
<210> 2
   <211> 495
   <212> PRT
   <213> Vigna angularis
<400> 2
   Met Val Leu Gln Thr Asn Asn Val Pro His Phe Val Leu Phe Pro Met
   1               5                   10                  15
   Met Ala Gln Gly His Ile Ile Pro Met Met Asp Ile Ala Arg Ile Leu
               20                  25                  30
   Ala Gln Arg Gly Val Ile Ile Thr Val Phe Thr Thr Pro Lys Asn Ala
           35                  40                  45
   Ser Arg Phe Asn Ser Val Ile Ser Arg Ala Val Ser Ser Gly Leu Lys
       50                  55                  60
   Ile Arg Leu Val Gln Leu Asn Phe Pro Ser Lys Glu Ala Gly Leu Arg
   65 70 75 80
   Glu Gly Cys Glu Asn Leu Asp Met Val Ser Ser Asn Asp Met Ser Lys
   85 90 95
   Ile Phe Gln Val Ile His Met Pro Gln Lys Pro Ala Glu Glu Phe Phe
   100 105 110
   Gln Thr Leu Thr Pro Lys Pro Ser Cys Ile Ile Ser Asp Phe Cys Ile
   115 120 125
   Ala Trp Thr Leu Gln Leu Ala Glu Lys Tyr His Ile Pro Arg Val Ser
   130 135 140
   Phe His Gly Phe Ser Cys Phe Cys Leu His Cys Arg Tyr Val Ile His
   145 150 155 160
   Thr Ser Asp Phe Cys Arg Ser Ile Thr Ser Glu Ser Lys Tyr Phe Thr
   165 170 175
   Ile Pro Gly Ile Pro Asp Lys Ile Gln Val Thr Lys Glu Gln Leu Pro
   180 185 190
   Gly Ser Leu Ala Thr Asp Leu Asp Asp Phe Lys Asp Gln Val Arg Asp
   195 200 205
   Ala Glu Lys Lys Ser Tyr Gly Val Ile Val Asn Thr Phe Trp Arg Val
   210 215 220
   Gly Glu Gly Ile Cys Glu Gly Phe Ser Arg Arg Leu Lys Asn Asn Lys
   225 230 235 240
   Ala Trp Phe Ile Gly Pro Val Ser Leu Cys Asn Lys Asp Gly Leu Asp
   245 250 255
   Lys Ala Gln Arg Gly Lys Gln Ala Ser Ile Asn Glu Asn His Cys Leu
   260 265 270
   Lys Trp Leu Asp Val Gln Gln Ala Lys Ser Val Val Tyr Val Cys Phe
   275 280 285
   Gly Ser Ile Cys Asn Leu Ile Pro Ser Gln Leu Val Glu Leu Ala Leu
       290                 295                 300
   Ala Leu Glu Asp Thr Lys Arg Pro Phe Val Trp Val Ile Arg Glu Gly
   305                 310                 315                 320
   Ser Gln Leu Gln Glu Leu Glu Lys Trp Phe Ser Glu Glu Gly Phe Glu
                   325                 330                 335
   Glu Arg Thr Lys Gly Arg Gly Leu Ile Ile Gly Gly Trp Ala Pro Gln
               340                 345                 350
   Val Met Ile Leu Ser His Pro Ser Ile Gly Gly Phe Leu Thr His Cys
           355                 360                 365
   Gly Trp Asn Ser Thr Leu Glu Gly Ile Cys Ala Gly Val Pro Leu Val
       370                 375                 380
   Thr Trp Pro Leu Phe Gly Asp Gln Phe Leu Asn Glu Lys Pro Val Ser
   385                 390                 395                 400
   Asp Val Leu Arg Ile Gly Val Ser Val Gly Ala Glu Val Pro Leu Lys
                   405                 410                 415
   Trp Gly Glu Glu Glu Lys Arg Gly Val Met Val Lys Lys Asp Asp Ile
               420                 425                 430
   Lys Arg Ala Ile Cys Met Val Met Asp Asp Glu Glu Gly Lys Glu Arg
           435                 440                 445
   Arg Glu Arg Val Cys Lys Leu Ser Glu Met Gly Lys Arg Ala Val Glu
       450                 455                 460
   Glu Gly Gly Ser Ser His Leu Asp Val Thr Leu Leu Ile Gln Asp Ile
   465                 470                 475                 480
   Met Gln Gln Thr Asn Thr Lys Glu Asp Glu Ser Leu Ser Asp Val
                   485                 490                 495
<210> 3
   <211> 476
   <212> PRT
   <213> Nicotiana tabacum
<400> 3
   Met Gly Gln Leu His Phe Phe Phe Phe Pro Val Met Ala His Gly His
   1               5                   10                  15
   Met Ile Pro Thr Leu Asp Met Ala Lys Leu Phe Ala Ser Arg Gly Val
               20                  25                  30
   Lys Ala Thr Ile Ile Thr Thr Pro Leu Asn Glu Phe Val Phe Ser Lys
           35                  40                  45
   Ala Ile Gln Arg Asn Lys His Leu Gly Ile Glu Ile Glu Ile Arg Leu
       50                  55                  60
   Ile Lys Phe Pro Ala Val Glu Asn Gly Leu Pro Glu Glu Cys Glu Arg
   65                  70                  75                  80
   Leu Asp Gln Ile Pro Ser Asp Glu Lys Leu Pro Asn Phe Phe Lys Ala
                   85                  90                  95
   Val Ala Met Met Gln Glu Pro Leu Glu Gln Leu Ile Glu Glu Cys Arg
               100                 105                 110
   Pro Asp Cys Leu Ile Ser Asp Met Phe Leu Pro Trp Thr Thr Asp Thr
           115                 120                 125
   Ala Ala Lys Phe Asn Ile Pro Arg Ile Val Phe His Gly Thr Ser Phe
       130                 135                 140
   Phe Ala Leu Cys Val Glu Asn Ser Val Arg Leu Asn Lys Pro Phe Lys
   145                 150                 155                 160
   Asn Val Ser Ser Asp Ser Glu Thr Phe Val Val Pro Asp Leu Pro His
                   165                 170                 175
   Glu Ile Lys Leu Thr Arg Thr Gln Val Ser Pro Phe Glu Arg Ser Gly
               180                 185                 190
   Glu Glu Thr Ala Met Thr Arg Met Ile Lys Thr Val Arg Glu Ser Asp
           195                 200                 205
   Ser Lys Ser Tyr Gly Val Val Phe Asn Ser Phe Tyr Glu Leu Glu Thr
   210 215 220
   Asp Tyr Val Glu His Tyr Thr Lys Val Leu Gly Arg Arg Ala Trp Ala
   225 230 235 240
   Ile Gly Pro Leu Ser Met Cys Asn Arg Asp Ile Glu Asp Lys Ala Glu
   245 250 255
   Arg Gly Lys Lys Ser Ser Ile Asp Lys His Glu Cys Leu Lys Trp Leu
   260 265 270
   Asp Ser Lys Lys Pro Ser Ser Val Val Tyr Val Cys Phe Gly Ser Val
   275 280 285
   Ala Asn Phe Thr Ala Ser Gln Leu His Glu Leu Ala Met Gly Ile Glu
   290 295 300
   Ala Ser Gly Gln Glu Phe Ile Trp Val Val Arg Thr Glu Leu Asp Asn
   305 310 315 320
   Glu Asp Trp Leu Pro Glu Gly Phe Glu Glu Arg Thr Lys Glu Lys Gly
   325 330 335
   Leu Ile Ile Arg Gly Trp Ala Pro Gln Val Leu Ile Leu Asp His Glu
   340 345 350
   Ser Val Gly Ala Phe Val Thr His Cys Gly Trp Asn Ser Thr Leu Glu
   355 360 365
   Gly Val Ser Gly Gly Val Pro Met Val Thr Trp Pro Val Phe Ala Glu
   370 375 380
   Gln Phe Phe Asn Glu Lys Leu Val Thr Glu Val Leu Lys Thr Gly Ala
   385 390 395 400
   Gly Val Gly Ser Ile Gln Trp Lys Arg Ser Ala Ser Glu Gly Val Lys
   405 410 415
   Arg Glu Ala Ile Ala Lys Ala Ile Lys Arg Val Met Val Ser Glu Glu
   420 425 430
   Ala Asp Gly Phe Arg Asn Arg Ala Lys Ala Tyr Lys Glu Met Ala Arg
   435 440 445
   Lys Ala Ile Glu Glu Gly Gly Ser Ser Tyr Thr Gly Leu Thr Thr Leu
   450 455 460
   Leu Glu Asp Ile Ser Thr Tyr Ser Ser Thr Gly His
   465 470 475
<210> 4
   <211> 476
   <212> PRT
   <213> Nicotiana tabacum
<400> 4
   Met Gly Gln Leu His Ile Phe Phe Phe Pro Val Met Ala His Gly His
   1 5 10 15
   Met Ile Pro Thr Leu Asp Met Ala Lys Leu Phe Ala Ser Arg Gly Val
   20 25 30
   Lys Ala Thr Ile Ile Thr Thr Pro Leu Asn Glu Phe Val Phe Ser Lys
   35 40 45
   Ala Ile Gln Arg Asn Lys His Leu Gly Ile Glu Ile Glu Ile Arg Leu
   50 55 60
   Ile Lys Phe Pro Ala Val Glu Asn Gly Leu Pro Glu Glu Cys Glu Arg
   65 70 75 80
   Leu Asp Gln Ile Pro Ser Asp Glu Lys Leu Pro Asn Phe Phe Lys Ala
   85 90 95
   Val Ala Met Met Gln Glu Pro Leu Glu Gln Leu Ile Glu Glu Cys Arg
   100 105 110
   Pro Asp Cys Leu Ile Ser Asp Met Phe Leu Pro Trp Thr Thr Asp Thr
   115 120 125
   Ala Ala Lys Phe Asn Ile Pro Arg Ile Val Phe His Gly Thr Ser Phe
   130 135 140
   Phe Ala Leu Cys Val Glu Asn Ser Val Arg Leu Asn Lys Pro Phe Lys
   145 150 155 160
   Asn Val Ser Ser Asp Ser Glu Thr Phe Val Val Pro Asp Leu Pro His
   165 170 175
   Glu Ile Lys Leu Thr Arg Thr Gln Val Ser Pro Phe Glu Arg Ser Gly
   180 185 190
   Glu Glu Thr Ala Met Thr Arg Met Ile Lys Thr Val Arg Glu Ser Asp
   195 200 205
   Ser Lys Ser Tyr Gly Val Val Phe Asn Ser Phe Tyr Glu Leu Glu Thr
   210 215 220
   Asp Tyr Val Glu His Tyr Thr Lys Val Leu Gly Arg Arg Ala Trp Ala
   225 230 235 240
   Ile Gly Pro Leu Ser Met Cys Asn Arg Asp Ile Glu Asp Lys Ala Glu
   245 250 255
   Arg Gly Lys Lys Ser Ser Ile Asp Lys His Glu Cys Leu Lys Trp Leu
   260 265 270
   Asp Ser Lys Lys Pro Ser Ser Val Val Tyr Ile Cys Phe Gly Ser Val
   275 280 285
   Ala Asn Phe Thr Ala Ser Gln Leu His Glu Leu Ala Met Gly Val Glu
   290 295 300
   Ala Ser Gly Gln Glu Phe Ile Trp Val Val Arg Thr Glu Leu Asp Asn
   305 310 315 320
   Glu Asp Trp Leu Pro Glu Gly Phe Glu Glu Arg Thr Lys Glu Lys Gly
   325 330 335
   Leu Ile Ile Arg Gly Trp Ala Pro Gln Val Leu Ile Leu Asp His Glu
   340 345 350
   Ser Val Gly Ala Phe Val Thr His Cys Gly Trp Asn Ser Thr Leu Glu
   355 360 365
   Gly Val Ser Gly Gly Val Pro Met Val Thr Trp Pro Val Phe Ala Glu
   370 375 380
   Gln Phe Phe Asn Glu Lys Leu Val Thr Glu Val Leu Lys Thr Gly Ala
   385 390 395 400
   Gly Val Gly Ser Ile Gln Trp Lys Arg Ser Ala Ser Glu Gly Val Lys
   405 410 415
   Arg Glu Ala Ile Ala Lys Ala Ile Lys Arg Val Met Val Ser Glu Glu
   420 425 430
   Ala Asp Gly Phe Arg Asn Arg Ala Lys Ala Tyr Lys Glu Met Ala Arg
   435 440 445
   Lys Ala Ile Glu Glu Gly Gly Ser Ser Tyr Thr Gly Leu Thr Thr Leu
   450 455 460
   Leu Glu Asp Ile Ser Thr Tyr Ser Ser Thr Gly His
   465 470 475
<210> 5
   <211> 497
   <212> PRT
   <213> Oryza sativa
<400> 5
   Met Ser Ser Ala Gly His Ala Val Asp Gln Gln Arg Lys Ser Thr Thr
   1               5                   10                  15
   Met Lys Ala His Phe Val Leu Val Pro Met Met Ala Gln Gly His Met
               20                  25                  30
   Ile Pro Met Thr Gly Met Ala Arg Leu Leu Ala Glu His Gly Ala Gln
           35                  40                  45
   Val Ser Phe Val Thr Thr Pro Val Asn Ala Ala Arg Met Ala Gly Phe
       50                  55                  60
   Val Thr Ala Val Glu Ala Ala Gly Leu Ala Val Gln Leu Val Lys Leu
   65                  70                  75                  80
   Pro Phe Pro Ala Thr Glu Phe Gly Leu Pro Asp Gly Cys Glu Asn Leu
   85 90 95
   Asp Met Ile Gln Ser Arg Asp Leu Ser Arg Asn Phe Met Glu Ala Cys
   100 105 110
   Gly Ala Leu Arg Glu Pro Leu Thr Ala Arg Leu Arg Gln Leu Cys Pro
   115 120 125
   Pro Pro Ser Cys Ile Ile Ser Asp Met Val Gln Trp Trp Thr Gly Glu
   130 135 140
   Ile Ala Arg Glu Leu Gly Ile Pro Arg Leu Thr Phe Asp Gly Phe Cys
   145 150 155 160
   Thr Phe Ala Ser Leu Ala Arg Tyr Ile Ile Phe Arg Asp Lys Leu Leu
   165 170 175
   Asp Asn Val Ala Asp Glu Glu Ile Val Thr Phe Ser Gly Phe Pro Met
   180 185 190
   Leu Leu Glu Leu Pro Lys Ala Arg Cys Pro Gly Ser Leu Cys Val Pro
   195 200 205
   Gly Met Glu Gln Ile Arg Asp Lys Met Tyr Glu Glu Glu Leu Gln Ser
   210 215 220
   Asp Gly Asn Val Met Asn Ser Phe Gln Glu Leu Glu Thr Leu Tyr Ile
   225 230 235 240
   Glu Ser Phe Glu Gln Ile Thr Gly Lys Lys Val Trp Thr Ile Gly Pro
   245 250 255
   Met Cys Leu Cys Asp Arg Asp Ser Asn Met Met Ala Ala Arg Gly Asn
   260 265 270
   Lys Ala Ser Val Asp Glu Ala Lys Cys Leu Gln Trp Leu Asp Ser Lys
   275 280 285
   Lys Pro Gly Ser Val Ile Phe Val Ser Phe Gly Ser Leu Ala Ser Thr
   290 295 300
   Ala Pro Gln Gln Leu Val Glu Leu Gly Leu Gly Leu Glu Ala Ser Lys
   305 310 315 320
   Glu Pro Phe Ile Trp Val Ile Lys Ala Gly Asn Lys Phe Pro Glu Val
   325 330 335
   Glu Glu Trp Leu Ala Asp Gly Phe Glu Glu Arg Val Lys Asp Arg Gly
   340 345 350
   Met Ile Ile Arg Gly Trp Ala Pro Gln Val Met Ile Leu Trp His Gln
   355 360 365
   Ala Ile Gly Gly Phe Met Thr His Cys Gly Trp Asn Ser Thr Ile Glu
   370 375 380
   Gly Ile Cys Ala Gly Val Pro Met Ile Thr Trp Pro His Phe Ala Glu
   385 390 395 400
   Gln Phe Leu Asn Glu Lys Phe Val Val Asn Leu Leu Lys Ile Gly Leu
   405 410 415
   Glu Ile Gly Val Lys Gly Val Ala Gln Trp Gly Ser Glu His Lys Glu
   420 425 430
   Val Arg Val Thr Arg Asn Ala Val Glu Thr Ala Val Ser Thr Leu Met
   435 440 445
   Asn Asp Gly Glu Ala Ala Gln Glu Met Arg Met Arg Ala Lys Asp Leu
   450 455 460
   Gly Val Lys Ala Arg Arg Ala Leu Glu Glu Gly Gly Ser Ser Tyr Asp
   465 470 475 480
   Asn Ile Ser Leu Leu Ile Gln Glu Met Gly Asn Lys Gln Asn Ala Ser
   485 490 495
   Gly
<210> 6
   <211> 466
   <212> PRT
   <213> Lycopersicon esculentum
<400> 6
   His Phe Phe Phe Phe Pro Asp Asp Ala Gln Gly His Met Ile Pro Thr
   1 5 10 15
   Leu Asp Met Ala Asn Val Val Ala Cys Arg Gly Val Lys Ala Thr Ile
   20 25 30
   Ile Thr Thr Pro Leu Asn Glu Ser Val Phe Ser Lys Ala Ile Glu Arg
   35 40 45
   Asn Lys His Leu Gly Ile Glu Ile Asp Ile Arg Leu Leu Lys Phe Pro
   50 55 60
   Ala Lys Glu Asn Asp Leu Pro Glu Asp Cys Glu Arg Leu Asp Leu Val
   65 70 75 80
   Pro Ser Asp Asp Lys Leu Pro Asn Phe Leu Lys Ala Ala Ala Met Met
   85 90 95
   Lys Asp Glu Phe Glu Glu Leu Ile Gly Glu Cys Arg Pro Asp Cys Leu
   100 105 110
   Val Ser Asp Met Phe Leu Pro Trp Thr Thr Asp Ser Ala Ala Lys Phe
   115 120 125
   Ser Ile Pro Arg Ile Val Phe His Gly Thr Ser Tyr Phe Ala Leu Cys
   130 135 140
   Val Gly Asp Thr Ile Arg Arg Asn Lys Pro Phe Lys Asn Val Ser Ser
   145 150 155 160
   Asp Thr Glu Thr Phe Val Val Pro Asp Leu Pro His Glu Ile Arg Leu
   165 170 175
   Thr Arg Thr Gln Leu Ser Pro Phe Glu Gln Ser Asp Glu Glu Thr Gly
   180 185 190
   Met Ala Pro Met Ile Lys Ala Val Arg Glu Ser Asp Ala Lys Ser Tyr
   195 200 205
   Gly Val Ile Phe Asn Ser Phe Tyr Glu Leu Glu Ser Asp Tyr Val Glu
   210 215 220
   His Tyr Thr Lys Val Val Gly Arg Lys Asn Trp Ala Ile Gly Pro Leu
   225 230 235 240
   Ser Leu Cys Asn Arg Asp Ile Glu Asp Lys Ala Glu Arg Gly Arg Lys
   245 250 255
   Ser Ser Ile Asp Glu His Ala Cys Leu Lys Trp Leu Asp Ser Lys Lys
   260 265 270
   Ser Ser Ser Ile Val Tyr Val Cys Phe Gly Ser Thr Ala Asp Phe Thr
   275 280 285
   Thr Ala Gln Met Gln Glu Leu Ala Met Gly Leu Glu Ala Ser Gly Gln
   290 295 300
   Asp Phe Ile Trp Val Ile Arg Thr Gly Asn Glu Asp Trp Leu Pro Glu
   305 310 315 320
   Gly Phe Glu Glu Arg Thr Lys Glu Lys Gly Leu Ile Ile Arg Gly Trp
   325 330 335
   Ala Pro Gln Ser Val Ile Leu Asp His Glu Ala Ile Gly Ala Phe Val
   340 345 350
   Thr His Cys Gly Trp Asn Ser Thr Leu Glu Gly Ile Ser Ala Gly Val
   355 360 365
   Pro Met Val Thr Trp Pro Val Phe Ala Glu Gln Phe Phe Asn Glu Lys
   370 375 380
   Leu Val Thr Glu Val Met Arg Ser Gly Ala Gly Val Gly Ser Lys Gln
   385 390 395 400
   Trp Lys Arg Thr Ala Ser Glu Gly Val Lys Arg Glu Ala Ile Ala Lys
   405 410 415
   Ala Ile Lys Arg Val Met Ala Ser Glu Glu Thr Glu Gly Phe Arg Ser
   420 425 430
   Arg Ala Lys Glu Tyr Lys Glu Met Ala Arg Glu Ala Ile Glu Glu Gly
   435 440 445
   Gly Ser Ser Tyr Asn Gly Trp Ala Thr Leu Ile Gln Asp Ile Thr Ser
   450 455 460
   Tyr Arg
   465
<210> 7
   <211> 489
   <212> PRT
   <213> Dorothenathus bellidiformis
<400> 7
   Met Gly Thr His Ser Thr Ala Pro Asp Leu His Val Val Phe Phe Pro
   1 5 10 15
   Phe Leu Ala His Gly His Met Ile Pro Ser Leu Asp Ile Ala Lys Leu
   20 25 30
   Phe Ala Ala Arg Gly Val Lys Thr Thr Ile Ile Thr Thr Pro Leu Asn
   35 40 45
   Ala Ser Met Phe Thr Lys Ala Ile Glu Lys Thr Arg Lys Asn Thr Glu
   50 55 60
   Thr Gln Met Glu Ile Glu Val Phe Ser Phe Pro Ser Glu Glu Ala Gly
   65 70 75 80
   Leu Pro Leu Gly Cys Glu Asn Leu Glu Gln Ala Met Ala Ile Gly Ala
   85 90 95
   Asn Asn Glu Phe Phe Asn Ala Ala Asn Leu Leu Lys Glu Gln Leu Glu
   100 105 110
   Asn Phe Leu Val Lys Thr Arg Pro Asn Cys Leu Val Ala Asp Met Phe
   115 120 125
   Phe Thr Trp Ala Ala Asp Ser Thr Ala Lys Phe Asn Ile Pro Thr Leu
   130 135 140
   Val Phe His Gly Phe Ser Phe Phe Ala Gln Cys Ala Lys Glu Val Met
   145 150 155 160
   Trp Arg Tyr Lys Pro Tyr Lys Ala Val Ser Ser Asp Thr Glu Val Phe
                   165                 170                 175
   Ser Leu Pro Phe Leu Pro His Glu Val Lys Met Thr Arg Leu Gln Val
               180                 185                 190
   Pro Glu Ser Met Arg Lys Gly Glu Glu Thr His Phe Thr Lys Arg Thr
           195                 200                 205
   Glu Arg Ile Arg Glu Leu Glu Arg Lys Ser Tyr Gly Val Ile Val Asn
       210                 215                 220
   Ser Phe Tyr Glu Leu Glu Pro Asp Tyr Ala Asp Phe Leu Arg Lys Glu
   225                 230                 235                 240
   Leu Gly Arg Arg Ala Trp His Ile Gly Pro Val Ser Leu Cys Asn Arg
                   245                 250                 255
   Ser Ile Glu Asp Lys Ala Gln Arg Gly Arg Gln Thr Ser Ile Asp Glu
               260                 265                 270
   Asp Glu Cys Leu Lys Trp Leu Asn Ser Lys Lys Pro Asp Ser Val Ile
           275                 280                 285
   Tyr Ile Cys Phe Gly Ser Thr Gly His Leu Ile Ala Pro Gln Leu His
       290                 295                 300
   Glu Ile Ala Thr Ala Leu Glu Ala Ser Gly Gln Asp Phe Ile Trp Ala
   305                 310                 315                 320
   Val Arg Gly Asp His Gly Gln Gly Asn Ser Glu Glu Trp Leu Pro Pro
                   325                 330                 335
   Gly Tyr Glu His Arg Leu Gln Gly Lys Gly Leu Ile Ile Arg Gly Trp
               340                 345                 350
   Ala Pro Gln Val Leu Ile Leu Glu His Glu Ala Thr Gly Gly Phe Leu
           355                 360                 365
   Thr His Cys Gly Trp Asn Ser Ala Leu Glu Gly Ile Ser Ala Gly Val
       370                 375                 380
   Pro Met Val Thr Trp Pro Thr Phe Ala Glu Gln Phe His Asn Glu Gln
   385 390 395 400
   Leu Leu Thr Gln Ile Leu Lys Val Gly Val Ala Val Gly Ser Lys Lys
   405 410 415
   Trp Thr Leu Lys Pro Ser Ile Glu Asp Val Ile Lys Ala Glu Asp Ile
   420 425 430
   Glu Lys Ala Val Arg Glu Val Met Val Gly Glu Glu Gly Glu Glu Arg
   435 440 445
   Arg Arg Arg Ala Lys Lys Leu Lys Glu Met Ala Trp Arg Ala Ile Glu
   450 455 460
   Glu Gly Gly Ser Ser Tyr Ser Asp Leu Ser Ala Leu Ile Glu Glu Leu
   465 470 475 480
   Lys Gly Tyr His Thr Ser Glu Lys Glu
   485
<210> 8
   <211> 30
   <212> DNA
   <213> Artificial
<220>
   <223> Description of Artificial Sequence:DNA
<400> 8
   actaagcttg gaatcatggt ttccgaaaca 30
<210> 9
   <211> 25
   <212> DNA
   <213> Artificial
<220>
   <223> Description of Artificial Sequence:DNA
<400> 9
   aagcggccgc atactcaatt attgg 25
<210> 10
   <211> 31
   <212> DNA
   <213> Artificial
<220>
   <223> Description of Artificial Sequence:DNA
<400> 10
   ctaagcttgg aatcatggca tcggaatttc g 31
<210> 11
   <211> 30
   <212> DNA
   <213> Artificial
<220>
   <223> Description of Artificial Sequence:DNA
<400> 11
   tagcggccgc attcatttct tgggttgttc 30
<210> 12
   <211> 32
   <212> DNA
   <213> Artificial
<220>
   <223> Description of Artificial Sequence:DNA
<400> 12
   ctaagcttgg aatcatggct ttcgagaaga cc 32
<210> 13
   <211> 30
   <212> DNA
   <213> Artificial
<220>
   <223> Description of Artificial Sequence: DNA
<400> 13
   tagcggccgc attcaactct tggattctac 30
<210> 14
   <211> 32
   <212> DNA
   <213> Artificial
<220>
   <223> Description of Artificial Sequence:DNA
<400> 14
   ctaagcttgg aatcatggct acggaaaaaa cc 32
<210> 15
   <211> 29
   <212> DNA
   <213> Artificial
<220>
   <223> Description of Artificial Sequence:DNA
<400> 15
   tagcggccgc attcattctt gaattgtgc 29
<210> 16
   <211> 31
   <212> DNA
   <213> Artificial
<220>
   <223> Description of Artificial Sequence:DNA
<400> 16
   ctaagcttgg aatcatggct tccgaaaaat c 31
<210> 17
   <211> 29
   <212> DNA
   <213> Artificial
<220>
   <223> Description of Artificial Sequence:DNA
<400> 17
   tagcggccgc attcagttct tggatttca 29
<210> 18
   <211> 31
   <212> DNA
   <213> Artificial
<220>
   <223> Description of Artificial Sequence:DNA
<400> 18
   ctaagcttgg aacatgtgtt ctcatgatcc t 31
<210> 19
   <211> 30
   <212> DNA
   <213> Artificial
<220>
   <223> Description of Artificial Sequence:DNA
<400> 19
   tagcggccgc attcaattat tggactgtgc 30
<210> 20
   <211> 59
   <212> DNA
   <213> Artificial
<220>
   <223> Description of Artificial Sequence: DNA
<400> 20
   ggatgcccga acaaaagtta atttcagaag aggacttatc aaagcttgag gcctcgcga 59
<210> 21
   <211> 26
   <212> DNA
   <213> Artificial
<220>
   <223> Description of Artificial Sequence: DNA
<400> 21
   tacaagcgaa atcgccaaga agttca 26
<210> 22
   <211> 26
   <212> DNA
   <213> Artificial
<220>
   <223> Description of Artificial Sequence:DNA
<400> 22
   cttcttggcg atttcgcttg tataag 26
<210> 23
   <211> 30
   <212> DNA
   <213> Artificial
<220>
   <223> Description of Artificial Sequence:DNA
<400> 23
   actaagcttg gaatcatggt ttccgaaaca 30
<210> 24
   <211> 25
   <212> DNA
   <213> Artificial
<220>
   <223> Description of Artificial Sequence:DNA
<400> 24
   tcttgtgaat tcaactctat cagga 25
<210> 25
   <211> 27
   <212> DNA
   <213> Artificial
<220>
   <223> Description of Artificial Sequence:DNA
<400> 25
   tacaagcaaa atcgccaaga agttcaa 27
<210> 26
   <211> 25
   <212> DNA
   <213> Artificial
<220>
   <223> Description of Artificial Sequence:DNA
<400> 26
   acttcttggc gattttgctt gtatt 25
<210> 27
   <211> 31
   <212> DNA
   <213> Artificial
<220>
   <223> Description of Artificial Sequence: DNA
<400> 27
   taagcttgga atcatgtgtt ctcatgatcc t 31
<210> 28
   <211> 26
   <212> DNA
   <213> Artificial
<220>
   <223> Description of Artificial Sequence:DNA
<400> 28
   tcacccggga aacagtaatc atgtcc 26
<210> 29
   <211> 22
   <212> DNA
   <213> Artificial
<220>
   <223> Description of Artificial Sequence:DNA
<400> 29
   cgaggcagat cgtcagtcag tc 22
<210> 30
   <211> 19
   <212> DNA
   <213> Artificial
<220>
   <223> Description of Artificial Sequence:DNA
<400> 30
   atccggggtt gaacagcct 19
<210> 31
   <211> 19
   <212> DNA
   <213> Artificial
<220>
   <223> Description of Artificial Sequence:DNA
<400> 31
   tcaattattg ggttctgcc 19
<210> 32
   <211> 19
   <212> DNA
   <213> Artificial
<220>
   <223> Description of Artificial Sequence: DNA
<400> 32
   ggagaaaata ggagtgtta 19
<210> 33
   <211> 19
   <212> DNA
   <213> Artificial
<220>
   <223> Description of Artificial Sequence:DNA
<400> 33
   tcagttcttg gatttcact 19
<210> 34
   <211> 18
   <212> DNA
   <213> Artificial
<220>
   <223> Description of Artificial Sequence:DNA
<400> 34
   gagaaactgg tcgtacaa 18
<210> 35
   <211> 19
   <212> DNA
   <213> Artificial
<220>
   <223> Description of Artificial Sequence:DNA
<400> 35
   tcaattattg gactgtgct 19
<210> 36
   <211> 21
   <212> DNA
   <213> Artificial
<220>
   <223> Description of Artificial Sequence:DNA
<400> 36
   gtccttcttt ctggtaaacg t 21
<210> 37
   <211> 21
   <212> DNA
   <213> Artificial
<220>
   <223> Description of Artificial Sequence:DNA
<400> 37
   aacccttgag gttgaatcat c 21
<210> 38
   <211> 38
   <212> DNA
   <213> Artificial
<220>
   <223> Description of Artificial Sequence:DNA
<400> 38
   gttaaaagct tacatgtgca ttacggtctg tgtgaata 38
<210> 39
   <211> 38
   <212> DNA
   <213> Artificial
<220>
   <223> Description of Artificial Sequence:DNA
<400> 39
   tttcggatcc catgattcaa ccttagtaag aaactctc 38

## Description

The present invention relates to a method for detoxification of mycotoxins as well as a method for producing derivatives of mycotoxins.

A complex of closely related species of the genus Fusarium is responsible for destructive and economically very important diseases of cereal crops (Fusarium head blight, FHB, of wheat and barley) and maize (Fusarium ear rot). In years with climatic conditions that favor the development of the fungi, Fusarium infections can reach epidemic proportions (1). Diseases caused by the plant pathogens do not only severely reduce yield, but also result in contamination of grain with unacceptable high amounts of mycotoxins, a problem of world-wide significance. A toxin class of particular concern to human and animal health are trichothecenes, sesquiterpenoid epoxides which are potent inhibitors of eukaryotic protein synthesis. More than 180 compounds of this class have been isolated from natural sources, predominantly from Fusarium species (2). Depending on the concentration and the substitution pattern of the trichothecene, either translational initiation, elongation or termination are preferentially inhibited (3).

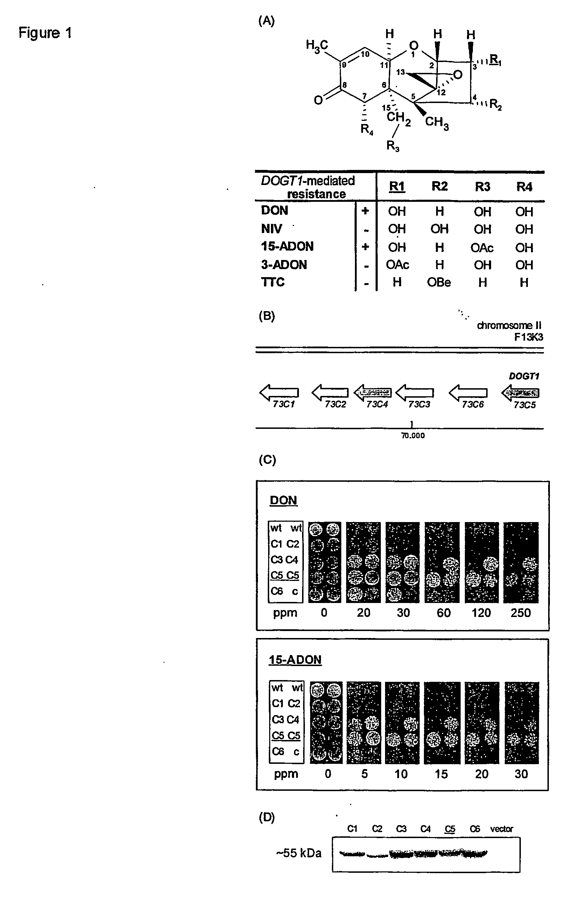

F. graminearum and F. culmorum are the two most relevant causative agents of FHB of cereals. Whereas F. graminearum lineages present in Europe and North America produce predominantly deoxynivalenol (DON), and the acetylated derivatives 3-acetyl-deoxynivalenol (3-ADON) and 15-acetyl-deoxynivalenol (15-ADON), producers of nivalenol (NIV), which contains one additional hydroxyl group (Figure 1A), dominate in Asia (4). While the toxicity of these trichothecenes is well studied in animal systems (5), little is known about differences in phytotoxicity. Animal exposure to DON (which is also known as vomitoxin) has been shown to cause adverse health effects, the neural and immune system being the most sensitive targets (6). In contrast to high doses of DON, which inhibit antibody production, low doses of DON act synergistically with bacterial lipopolysaccharide stimulating proinflamatory processes (6). To protect consumers, advisory levels for food have been established by the US Food and Drug Administration, and recently action levels for DON have been recommended by the European Community (7).

The role of DON in plant disease is still matter of discussion (8), but most of the evidence is in favor of a function as virulence factor. Fusarium mutants with a disrupted gene involved in trichothecene biosynthesis (trichodiene synthase, Tri5) are still pathogenic, however they exhibit reduced virulence on wheat (9) due to their inability to spread from the infection site (10). DON can move ahead of the fungus in infested plants, suggesting a role in conditioning host tissue for colonization (11). Results of several studies indicate that the in vitro resistance of wheat cultivars towards DON correlates with FHB resistance in the field (12), which is also the case for segregating experimental populations.

Increased resistance against toxic substances can be caused by several mechanisms, ranging from reduced uptake to bypass, overexpression or mutation of the toxin target. Yet, a very prominent process seems to be metabolic transformation often followed by compartmentation (13). An observed decline in the concentration of DON, which occurred in Fusarium infected wheat in the field (14) suggested that the toxin may be metabolized.

Acetylation of trichothecenes (e.g. DON) was suggested as a potential resistance strategy (US 6,346,655 B1), however, the resulting acetylated trichothecenes (e.g. Acetyl-DON = ADON) is equivalent in its overall toxicity to the non-acetylated form (e.g. DON), as proven by Eudes et al. in a coleoptile elongation test (53). Therefore, acetylation does not result in a reduction of trichothecene toxicity.

Two wheat cultivars differing in Fusarium resistance showed differences in their ability to form a DON metabolite, which was suspected to be a glucoside (15). Sewald et al. (16) incubated maize suspension culture with radiolabeled DON and proved that it was primarily conjugated to 3-β-D-glucopyranosyl-4-deoxynivalenol. No plant enzymes capable of modifying the trichothecenes or other mycotoxins by transfer of a sugar moiety have been described so far.

Genome sequencing projects revealed the existence of a vast number of genes in plants that code for putative UDP-glycosyltransferases (UGT), predicted to conjugate small molecules. For instance, Arabidopsis thaliana has been shown to harbor more than 100 members of this multigene family (17) whose functions are largely unknown.

The WO 98/34471 discloses a DNA sequence encoding for solanidine UDP-glucose glucosyltransferase (SGT). This DNA sequence may be used to identify inhibitors, in particular antisense inhibitors which may be applied on solanidine producing plants like potatoes in order to inhibit biosynthesis of SGT and consequently the production of solanidine.

In the WO 97/45546 a gene is disclosed which encodes for a glucosyltransferase which is responsible for the induction of plant defence and resistance responses as well as regulating plant development events.

The WO 00/00626 relates to a method for the enzymatic glucosylation of specific aromatic derivatives such as coumarin.

In the scientific publication of Von Rad U. et al. (Plant Journal (2001), 28:633-642) the characterisation of two glucosyltransferases which are involved in the detoxification of benzoxazinoids in maize are described. Due to a glucosylation of these substances the autotoxicity of the plant can be reduced because glucosylated products show a reduced chemical reactivity.

In Yamazaki M. et al. (J. Biol. Chem (1999)274:7405-7411) the characterisation of a UDP-glucose glucosyltransferase is described which is able to glucolise anthocyanin synthesised in plants.

In Eng-Kiat L. et al. (Biochem. J. (2003)373:987-992) glucosyltransferases are disclosed which can be heterogously expressed in Arabidopsis plant cells which may be used for glucosylating caffeic acid.

In Jackson R.G. et al. (Plant. J. (2002):473-583) the characterisation of a glucosyl transferase of Arabidopsis thaliana is described which is able to glucosylate in vitro indole-3-acetic acid.

In Yamazaki M. et al. (Plant. Mol. Biol. (2002) 48:401-411) the cloning and glucosylation of two flavonoid glucosyltransferases of the plant Petunia hybrida is described. These glucosyl transferases are responsible for the glucosylation of anthocyanides.

In Okubara P.A. et al. (Theor Appl Genet (2002)106:74-83) an acetyltransferase of a fusarium strain is described which is able to reduce the toxicity of deoxynivalenol (DON) by transferring an acetyl group on said compound.

In Sewald N. et al. (Tetrahedron Asymmetry (1992) 3:953-960) the isolation of glucosylated desoxynivalenol from Zea Mays is disclosed which represents the main metabolite of desoxynivalenol in maize cells.

In Meßner B. et al. (Planta (2003) 217:138-146) a UDP-glucose-dependent glucosyltransferase of Arabidopsis thaliana is disclosed. This glucosyltransferases are able to reduce the toxicity of xenobiotics in a plant as well as in vitro.

In Miller J.D. et al. (Canad. J. Plant. Pathol. (1996) 8:147-150) the incubation of deoxynivalenol in a suspension of fusarium resistant wheat is described wherein it was discovered that in the course of said incubation deoxynivalenin is in part glucosylated.

In Gorst-Allman C.P. et al. (J. Chem. Soc. Perkin Trans I (1985) 7:1553-1556) the elucidation of trichothecene glycosides in with fusarium infected maize plants is described. The sugar residue of said glycosides was identified as being glucose.

Schäffner A. et al. (Acta Biotechnol. (2002) 22:141-152) describe general detoxification pathways in plants in order to protect the plant from endogene and xenobiotic substances.

It is therefore an object of the present invention to provide a method for detoxification of mycotoxins.

Suitable preparations of mycotoxins or derivatives thereof, especially sugar-conjugate derivatives of mycotoxins are often difficult to provide. Since stereoselectivity is crucial to this kind of products, purely chemical syntheses based on common bulk mycotoxins are often diffcult, if not impossible, at least on an industrial scale. Another object of the present invention is therefore to provide methods for synthesising sugar-conjugate derivatives of mycotoxins.

Therefore the present invention provides a method for detoxification of mycotoxins wherein a mycotoxin is contacted with a glucosyltransferase in the presence of an activated glucose.

As mentioned above, there has not been any report being published in the prior art with respect to (plant) enzymes being capable of modifying mycotoxins by transfer of a sugar molecule, and of course not with respect to such enzymes being able to detoxify such mycotoxins, i.e. reducing the toxicity of mycotoxins to a considerable extent, e.g. by more than 80 %, preferably more than 90 %, especially more than 95 %, as determined by specific mycotoxin assays, such as inhibition of in vitro protein synthesis.

According to the present invention it has been found that glucosyltransferases are very specific tools for detoxification of mycotoxins. Specificity is on the one hand given by a certain specificity of given glucosyltransferases to only a limited number of mycotoxins and on the other hand also specificity with respect to the mode of detoxification, namely by site specific glucosylation of the mycotoxins. While this specificity limits the scope of application of a given single glucosyltransferase, the present invention provides tools for easily determining the specificities of any glucosyltransferase with respect to any mycotoxin, especially trichothecene mycotoxins. Therefore, if a given enzyme is specific for a given set of mycotoxins, the present invention allows straightforward identification of specificities for other glucosyltransferases and mycotoxins.

Since uridine diphosphate glucose (UDP-glucose, e.g. glucose which is produced by enzymes, such as pyrophosphorylases) is a preferred activated biological form of glucose, a preferred embodiment of the present method uses UDP-glucosyltransferase as glucosyltransferase and an UDP-glucose as activated glucose (=cosubstrate), however, any form glucose, which can be utilised by a glucosyltransferase (e.g. ADP-glucose, CDP-glucose, or any other form of biologically and synthetically activated glucose being suitable substrates for transfer to mycotoxins by glucosyltransferases) can be used according to the present invention.

A glucosyltranferase according to the present invention is defined as a (naturally) occuring or synthetically (recombinantly) designed or produced enzyme which is capable (as its main or as a side activity) of transferring a glucose moiety to a substrate molecule using an activated glucose as co-substrate thereby producing a glucosylated substrate molecule.

Preferred mycotoxins to be detoxified by the method according to the present invention are - due to their economic importance - trichothecenes, especially trichothecenes having a free hydroxy-group at position C₃ and two hydrogen groups at C₄. Especially the latter trichothecenes are specifically glucosylated either at their position 3 or - if present - at a free hydroxy group at position 7 or at both positions. Therefore, preferred mycotoxins are deoxynivalenol, 15-acetyl-deoxynivalenol, 15-acetoxyscirpendiol or mixtures thereof.

A preferred way of performing the method according to the present invention is the in vivo mode. This can be done using recombinant DNA technology and/or glucosyltransferase expression enhancing compounds. Preferred examples comprise expression of a glucosyltransferase in a transgenic plant cell (or plant tissue or whole plant) containing a recombinant glucosyltransferase and/or a recombinant regulating region for a glucosyltransferase, especially a mycotoxin-inducible promoter, especially of a glucosyltransferase. Preferred examples for glucosyltransferase expression enhancing compounds are substances which up-regulate glucosyltransferases, e.g. toxins (if expression of the transferase is controlled by toxin-inducible promoters). In principle, any cell which can be genetically manipulated so that it expresses a recombinant (i.e. a gene which has been artificially introduced into this cell or its precursor and which naturally is not present at this site of the genome of the cell) plant glucosyltransferase gene can be used for this purpose. Preferred embodiments are transgenic bacteria, yeasts, baculovirus infected insect cells, etc. expressing a plant glucosyltransferase gene.

In an industrial process for detoxification of mycotoxins, the glucosyltransferase can advantageously be immobilised (according to standard immobilisation techniques) to a solid surface and a mycotoxin containing solution or suspension can then be contacted with this immobilised glucosyltransferase.

Preferred examples of glucosyltransferases to be used according to the present invention are UDP-glucosyltransferases corresponding to subfamily 73C of Arabidopsis thaliana, especially UDP-glucosyltransferases 73C4 and 73C5.

According to another aspect, the present invention enables a method for producing glucosylated mycotoxins wherein a mycotoxin is contacted with a glucosyltransferase in the presence of an activated glucose. Therewith, the above mentioned enzyme specificities can be properly used for producing specifically glucosylated mycotoxin derivatives. If a mycotoxin is glucosylated in a non-enzymatic ("chemical") way, a crude mixture of glucosylation products is obtained (each free hydroxy group is in principle an acceptor site for glucosyl residues). Acetylation as protecting means for such groups has also its drawbacks with respect to specificity (also all hydroxy groups are acetylated). In using substrate and site specificity, the present invention provides a method for producing well defined glucosylated derivatives. If more than one hydroxy group is glucosylated or if more than one position is capable of being glucosylated by a given glucosyltransferase, such a limited number of products (e.g. 2 to 5) may easily be separated by suitable separation means such as HPLC (for example, DON, being glucosylated at position number 3, is easily separatable from DON, being glucosylated at position 7).

The preferred method steps described above are also applicable for this aspect of the invention.

According to another aspect, the present invention relates to transgenic plants or a transgenic (plant) cells containing a recombinant glucosyltransferase and/or a recombinant expression regulating element, especially a promoter region for a glucosyltransferase. These plants or (plant) cells may be used in a method according to the present invention.

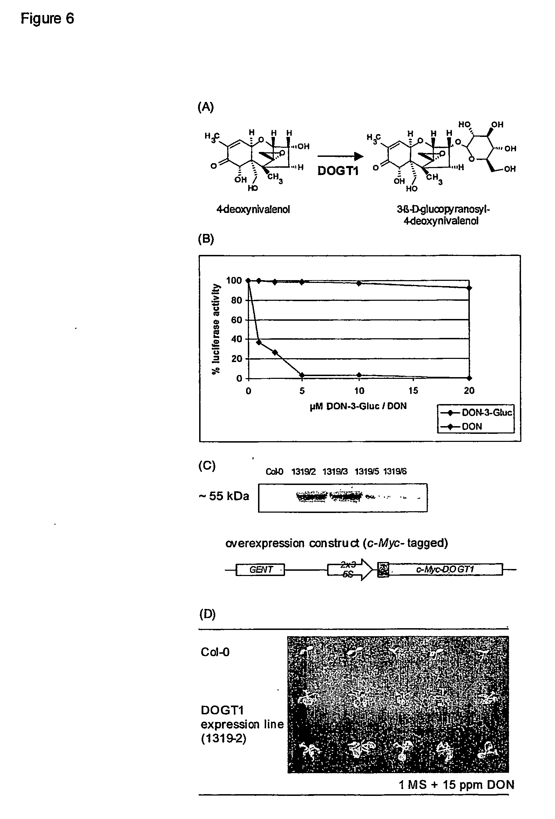

The present invention also relates to a genetically modified cell or organism, especially a plant or plant cell, comprising a non-naturally occurring (transgenic) glucosyltransferase in its genome and being mycotoxin resistant, especially trichothecene resistant, due to the presence of such a glucosyltransferase as transgene. Alternatively, an endogeneous glucosyltransferase in a cell may be subjected to a different, transgenic expression regulating region or element, such as a promoter, leading to an enhanced expression of the endogeneous glucosyltransferase (i.e. an enhanced expression activity of a homologous glucosyltransferase due to transgenic promoters). Also such cells show enhanced resistance against mycotoxins as described herein. Such cells and plants can be produced by applying standard methods to the teachings of the present invention (see e.g. Fig. 6B and the examples as well as e.g. (54)).

Preferred cells according to the present invention are also yeast cells. Since yeast cells may have a limited uptake capability, preferred yeast cells according to the present invention are designed or selected for an increased mycotoxin-uptake capability compared to the wild type or starting strain.

The genetically modified cells comprise one or more, preferably three or more, ABC transporters reduced or inactivated in their activity, especially pdr5. Such ABC transporter mutants are characterized by an enhanced uptake of mycotoxins. Preferably, the cells according to the present invention have a deletion in pdr5, pdr10, pdr15, snq2, yor1, ayt1 or combinations of these deletions.

These cells may preferably be used for detoxification of mycotoxin containing solutions or suspensions, especially for detoxification of trichothecenes in agriculture and beer production; and in the production of glucosylated mycotoxins, especially glucosylated DON or 15-ADON.

The present invention is further illustrated by the following examples and the figures, yet without being restricted thereto.

### Figures:

FIG. 1. Spectrum of trichothecene resistance of yeast expressing Arabidopsis thaliana UDP-glucosyltransferases of subfamily UGT73C. A, Structure and numbering of the ring system of class B trichothecenes used for resistance testing. The different residues at variable positions R1-R4 are indicated in the table (OAc: -OCOCH3, OBe: -OCOCH=CHCH3 (Z)). The relevant R1 is underlined (OH in DON, already blocked by acetylation in 3-ADON). Increased resistance conferred by DOGT1 expression is indicated by plus, lack of protection by minus. B, Genomic organisation of the UGT gene cluster on chromosome II (subfamily 73C) containing DOGT1 (UGT73C5, locus At2g36800). The DON detoxifying DOGT1 and 73C4 are shaded in gray. C, Yeast strains were spotted on YPD plates containing the indicated amount of toxin (ppm: mg/l). The strains are wild-type YZGA515 without plasmid (wt), this strain transformed with empty vector (c), and the transformants expressing the myc-tagged 73C UGTs (C5=DOGT1, underlined, spotted in duplicate). Resistance is conferred by plasmids leading to overexpression of C5 and C4. D, Western blot analysis of the yeast strains used for resistance testing. The N-terminal c-Myc epitope-tag introduced into the respective 73C UGT family members is detected (C5=DOGT1, underlined). A transformant containing the empty expression vector was used as control.

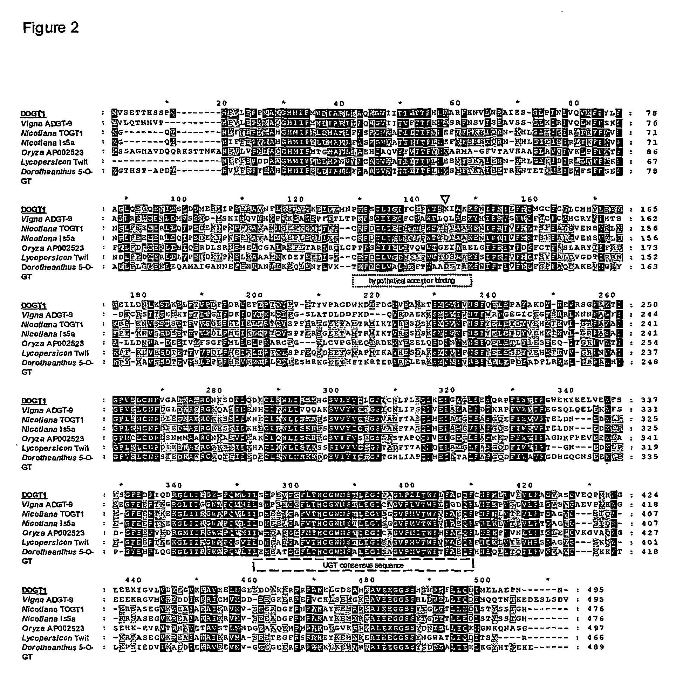

FIG. 2. Amino acid alignment of plant UDP-glucosyltransferases with high amino acid similarity to DOGT1. The regions implicated (Ref. 38) in acceptor substrate binding (dottet) and the UGT consensus sequence motif (dashed) are indicated by boxes below the sequences. The triangle above the sequence in the hypothetical acceptor binding region marks the lysine 136 in DOGT1 which has been altered by in vitro mutagenesis. The genbank accession numbers of the predicted proteins are: ADGT-9, glucosyltransferase-9 of Vigna angularis (AB070752); TOGT1, Phenylpropanoid:glucosyltransferase 1 of Nicotiana tabacum (AF346431); IS5a of Nicotiana tabacum (U32644); putative glucosyltransferase of Oryza sativa (AP002523); Twil of Lycopersicon esculentum (X85138); Betanidin-5-O-glucosyltransferase of Dorothenathus bellidiformis (Y18871).

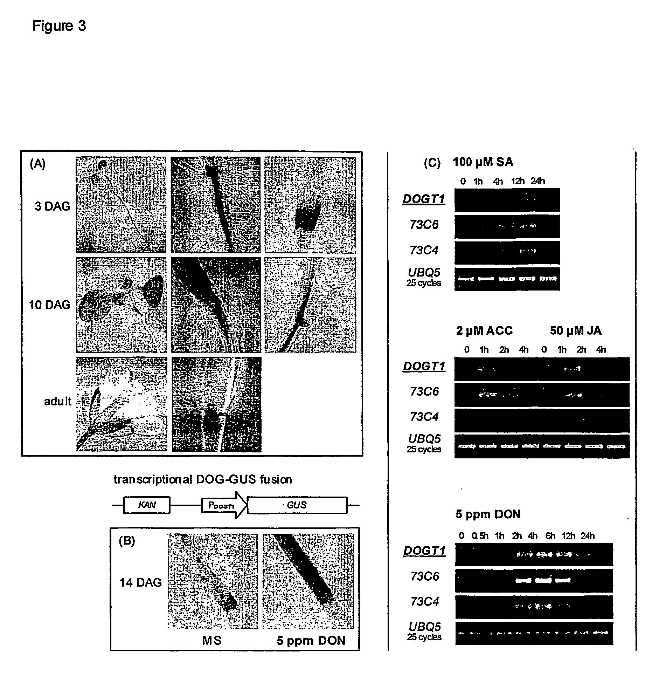

FIG. 3. DOGT1 expression is developmentally regulated and induced by DON and stress response related compounds. A, GUS-staining of seedlings homozygous for a transcriptional DOGT1 promoter-GUS fusion. Upper row: three days after germination (3 DAG) the expression of the fusion protein is restricted to the vasculature of root and hypocotyl and the meristematic region of the root tip. Middle: Later in development (10 DAG) staining in the root vasculature diminishes except for regions where lateral roots are formed. Lower row: In aerial parts of adult plants DOGT1 expression is restricted to petals of flowers and abscission zones. B, DON treatment (5 ppm for 4 h) of seedlings (14 DAG) expressing the translational GUS-fusion induces expression of the GUS reporter. Both samples were stained for 2 hours. C, Semiquantitative reverse transcriptase PCR analysis of induction of expression of DOGT1, UGT73C4 and UGT73C6 following treatment with DON (5 ppm), SA (100 µM), JA (50 µM) and ACC (2 µM). UBQ5 was used as an internal control.

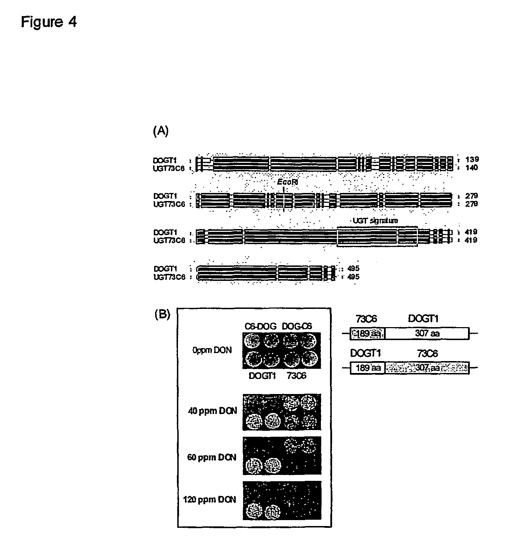

FIG. 4. The N-terminal part of DOGT1 is essential for its ability to detoxify DON. A, Amino acid alignment of DOGT1 and its closest homologue UGT73C6, which is not protecting against DON (black: amino acids identity). The conserved EcoRI restriction site was used to generate chimaeric UGTs consisting of the N-terminus of one and the C-terminus of the respective other gene. B, Yeast transformants were spotted in duplicate on YPD medium containing the indicated concentrations of DON. Upper left: transformants expressing the chimaeric enzyme consisting of the UGT73C6 N-terminus and DOGT1 C-terminus (short: C6-DOG). Upper right: Strains expressing the chimaeric protein consisting of DOGT1 N-terminus and UGT73C6 C-terminus (DOG-C6). Lower row: transformants expressing resistance conferring DOGT1 (left), and inactive 73C6 (right). The hybrid containing the N-terminal part of DOGT1 is resistant to higher DON concentrations than the strain expressing the other hybrid (C6-DOG) or UGT73C6.

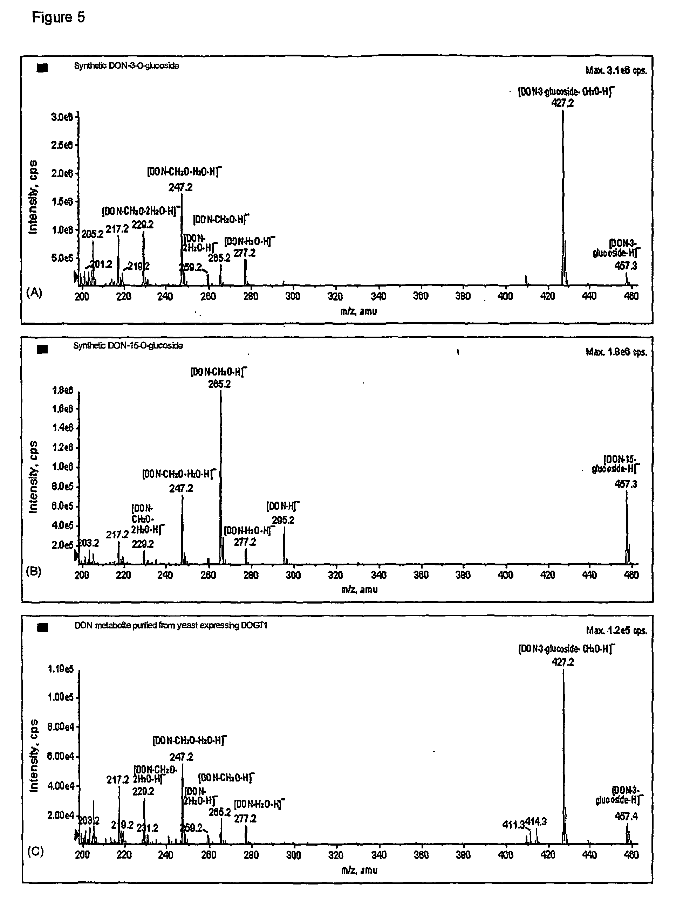

FIG. 5. Fragmentation pattern of synthesized DON-glucosides and a DON-metabolite formed in yeast cells expressing DOGT1 in mass spectrometry. A, The synthesized DON-3-O-glucoside yields a fragment of 427.2 m/z in the linear ion trap (MS/ MS/ MS). B, this fragment was not produced by the synthesized DON-15-O-glucoside sample as a separation of the -CH₂OH group at C₆ is prevented by the glucose moiety present at the hydroxyl group. C, The DON-metabolite formed in yeast expressing DOGT1 eluted in the HPLC with the same retention time as the DON-3-O-glucoside and showed the fragmentation pattern corresponding to the DON-3-O-glucosid reference substance.
FIG. 6. glucosylation of DON reduces toxicity in vitro and in vivo. A, Structure of the proposed reaction product: DOGT1 catalyses the transfer of glucose from UDP-glucose to the 3-OH position of DON. B, Comparison of the inhibition of in vitro protein synthesis of wheat ribosomes by DON and DON-3-O-glucoside, determined using a wheat germ extract based coupled transcription/translation system. Luminescence was measured and expressed as % luciferase activity of control samples without toxin. C, Western blot analysis of A. thaliana lines homozygous for an overexpression construct encoding c-Myc-tagged DOGT1. A schematic drawing of the plant transformation vector is shown below (GENT: gentamycin resistance used as selectable marker; 2x35S duplicated promoter of cauliflower 35S transcript (see Experimental Procedures for details)). Col-0 was used as a negative control. 2 lines with high and 2 lines with low levels of c-Myc-DOG1 protein are shown. D, Seed germination on DON containing MS medium. Compared to wild-type (Col-0) transgenic Arabidopsis lines expressing high amounts of DOGT1 (e.g. line 1319/2) exhibit enhanced resistance.

### EXAMPLES

For illustrating the present invention, in the examples hereinafter the cloning of an Arabidopsis UGT by functional expression in yeast and its characterization is described. This enzyme is able to detoxify the Fusarium mycotoxin DON and its acetylated derivative 15-ADON. The methods used in these examples are also directly applicable to other glucosyltransferases and mycotoxins without undue experimental burden.

### EXPERIMENTAL PROCEDURES

**Yeast strains.** The yeast strains used in this work are derived from YPH499 (Mat a, ade2-101oc, his3-Δ200, leu2-Δ 1, lys2-801a, trp1- Δ 1, ura3-52) (18). The relevant genotype of YZGA452 is pdr5Δ::TRP1, pdr10Δ::hisG, snq2Δ::hisG, yor1Δ::hisG. YZGA515 (pdr5Δ::TRP1, pdr10Δ::hisG, pdr15Δ::loxP-KanMX-loxP, ayt1Δ::URA3) was constructed by disruption of the acetyltransferase AYT1 in strain YHW10515.

**Plant material and growth conditions.** A. thaliana experiments were conducted with the wild-type ecotype Columbia-0 (Col-0). For propagation seeds were sterilized, plated on standard MS growth medium (19) supplemented with 1.0 % sucrose and 1.0% phytagar (Life Technologies) and subjected to a 2 day dark treatment at 4°C to synchronize germination. The seedlings were grown for 2 weeks in a controlled environment of 16h/8h light-dark cycle (140 µmol m⁻² sec⁻¹ white light) at 22°C before they were transferred to soil and grown at 20°C and 55% humidity under continuous white light.

**Arabidopsis thaliana cDNA library screen in yeast.** The ATP-binding cassette (ABC) transporter deficient Saccharomyces cerevisiae strain YZGA452, which is hypersensitive to DON, was transformed with an A. thaliana cDNA library constitutively expressed under the control of the phoshoglucerate kinase (PGK1) promoter (20). A total of 10⁷ transformants were selected on minimal medium lacking uracil and transferred to media containing 180 ppm DON, sufficient to completely inhibit growth of yeast transformed with the empty library plasmid. Colonies that showed resistance were isolated and from candidates that formed single colonies on toxin containing media, the plasmid dependency of the phenotype was tested by plasmid DNA preparation and retransformation of YZGA452. The NotI fragment containing the cDNA insert of the candidate (which was named DON glucosyltransferase 1, DOGT1) was subcloned into pBluescript SKII+ (Stratagene) and sequenced.

**Constitutive expression and immunodetection of the DON-glucosyltransferase (DOGT1) and close homologues in yeast.** The intron-less open reading frames (ORFs) of DOGT1 (UGT73C5, locus At2g36800) and 5 of its closest homologues (UGT73C1, At2g36750; UGT73C2, At2g36760; UGT73C3, At2g36780; UGT73C4, At2g36770; UGT73C6, At2g36790) were PCR amplified (Triple Master PCR System, Eppendorf) from genomic DNA using gene specific primers containing flanking HindIII and NotI restriction sites at the 5' and 3' ends, respectively
(DOGT1, fw: 5'-ACTAAGCTTGGAATCATGGTTTCCGAAACA-3', rv: 5'-AAGCGGCCGCATACTCAATTATTGG-3'; 73Cl, fw: 5'-CTAAGCTTGGAAT-CATGGCATCGGAATTTCG-3', rv: 5'-TAGCGGCCGCATTCATTTCTTGGGTTGTTC-3'; 73C2, fw: 5'-CTAAGCTTGGAATCATGGCTTTCGAGAAGACC-3', rv: 5'-TAGCG-GCCGCATTCAACTCTTGGATTCTAC-3';
73C3, fw: 5'-CTAAGCTTGGAATCATGGCTACGGAAAAAACC-3', rv: 5'-TAGCG-GCCGCATTCATTCTTGAATTGTGC-3';
73C4, fw: 5'-CTAAGCTTGGAATCATGGCTTCCGAAAAATC-3', rv: 5'-TAGCGGC-CGCATTCAGTTCTTGGATTTCA-3';
73C6: 5'-CTAAGCTTGGAACATGTGTTCTCATGATCCT-3', rv: 5'-TAGCGGCCGC-ATTCAATTATTGGACTGTGC-3'). The PCR products were cloned into the HindIII + NotI cloning sites of the yeast expression vector pYAK7 (PADH1-c-Myc-PDR5 LEU2 2µ), replacing the PDR5 gene. The vector pYAK7 was constructed by first inserting the double stranded linker 5'-GGATGCCCGAACAAAAGTTAATTTCAGAAGAGGACTTAT-CAAAGCTTGAGGCCTCGCGA into the SmaI site of vector pAD4Δ (21), thereby generating the N-terminal c-Myc epitope and a HindIII site, into which a genomic HindIII fragment containing the yeast PDR5 was inserted in frame.

The tagged UGT constructs were verified by sequencing and used to transform the yeast strain YZGA515. The empty vector (HindIII + NotI digested and religated pYAK7) was used as a control. Transformants were selected on minimal media lacking leucine. Exponentially growing cultures were diluted to OD600 0.05 and spotted on YPD media containing increasing concentrations of different trichothecenes. The toxins used were: DON, 3-ADON, 15-ADON, NIV, trichothecin (TTC), T-2 toxin, HT-2 toxin, diacetoxy-scirpenol (DAS) and veruccarin A (VA). With the exception of DON, 3-ADON and NIV, which were obtained from Biopure Referenz-substanzen GmbH (Tulln, Austria), mycotoxins were purchased from Sigma and stored at -20°C dissolved in 70 % ethanol.

For immunodetection, the extraction of proteins from yeast cells was performed as described by Egner et al. (22). Western blot analysis was conducted with a primary mouse anti c-Myc antibody (1:5000, clone 9E10, Invitrogen).

**Domain shuffling.** The ORFs including the N-terminal c-Myc tag of DOGT1 and its closest homologue UGT73C6 were isolated from the yeast expression vectors by SmaI + NotI digestion, cloned into vector pBluescript SKII+ (which was cut with XhoI and treated with Klenow enzyme, and subsequently digested with NotI). The resulting plasmids were digested with HindIII and a conserved EcoRI site present in both genes that cleaves DOGT1 at nucleotide position 565 (73C6 at 568). Hybrids were constructed by ligation of the N-terminal part of one gene to the C-terminal part of the other. The resulting genes were moved back into the yeast expression vector pYAK7 using the HindIII and NotI restriction sites. Yeast strain YZGA515 was used as a host to test the constructs for altered detoxification abilities.

**Site directed mutagenesis.** Mutations were constructed by overlap extension PCR (23) using overlapping mutant primers DOG-K136E-fw (5'-TACAAGCGAAATCGCCAAGAAGTTCA-3') and DOG-K136E-rv (5'-CTTCTTG-GCGATTTCGCTTGTATAAG-3') and flanking primers DOGIpYAK7-fw-a (5'-ACTAAGCTTGGAATCATGGTTTCCGAAACA-3') and DOG-EcoRI-rv (5'-TCT-TGTGAATTCAACTCTATC AGGA-3') for mutagenesis of DOGT1, and mutant primers 73C6-E1.37K-fw (5'-TACAAGCAAAATCGCC AAGAAGTTCAA-3') and 73C6-E137K-rv (5'-ACTTCTTGGCGATTTTGCTTGTATT-3') and flanking primers 73C6pYAK7-fw (5'-TAAGCTTGGAATCATGTGTTCTCATGATCCT-3') and DOG-EcoRI-rv for 73C6 mutagenesis. The resulting PCR products were cloned as HindIII+EcoRI fragments into the corresponding genes present in vector pBluescript SKII+. After sequencing, the ORFs were moved as HindIII + NotI fragments back into the yeast expression vector pYAK7 (replacing the PDR5 gene) and the resulting plasmids were transformed into strain YZGA515 to analyze the detoxification properties of the engineered UGTs.

**Synthesis of 3-β-D-Glucopyranosyl-4-deoxynivalenol and 15-β-D-Glucopyranosyl-4-deoxynivalenol.** To obtain reference material for HPLC and TLC, DON-3-glucoside and DON-15-glucoside were synthesized in 2-step reactions. In the first step, 15-Acetyl-DON or 3-Acetyl-DON were modified with 1ββ-Bromo-1-deoxy-2,3,4,6-tetra-O-acetyl-α-D-glucopyranose (acetobromoglucose) in toluene with CdCO₃ as catalyst to yield the DON-glucoside-acetates (24). Gentle hydrolysis of the acetates to the glucosides was performed in the second step using a strong basic anion exchanger (DOWEX1x2-400, Aldrich, USA). After checking the progress of the reaction with TLC (mobile phase toluene/ethyl acetate 1:1, v/v), the DON-glucosides were cleaned up using flash chromatography over silica gel with 1-butanol/1-propanol/ethanol/water (2:3:3:1, v/v/v/v). Further purification of the substances was performed with HPLC, by means of a RP-18 Aquasil column (Keystone, Waltham, USA) using acetonitrile/water (10:90, v/v) at 22°C.

The synthesized DON-derivatives were characterized in the negative electrospray interface (ESI) mode. LC-MS/MS analysis was performed on a QTrap-LC-MS/MS system (Applied Biosystems, Foster City, USA) equipped with ESI and a 1100 Series HPLC system (Agilent, Waldbronn, Germany). Chromatographic separation was achieved on a 150 mm x 4,6 mm i.d., 3 µm, Aquasil RP-18 column (Keystone, Waltham, USA) at 22°C using methanol/water (28:72, v/v). The flow rate was set to 0.3 ml/min. The ESI interface was used in the negative ion mode at 400°C with CUR 20 psi, GS1 30 psi, GS2 75 psi, IS -4200 V, DP -46 V, EP -9 V, CE -30 eV, CAD high, LIT fill time 50 ms, Q3 entry barrier 8 V.

**Isolation and analysis of DON metabolites in vivo.** To elucidate the chemical structure of DON metabolites that result from enzymatic transformation of the mycotoxin by DOGT1, a highly tolerant strain was constructed and the DON-metabolite was extracted from toxin treated cells for subsequent HPLC analysis.

The yeast strain YZGA515 was transformed with c-Myc-tagged DOGT1 under the control of the constitutive ADH1 promoter and in addition with plasmid pRM561. This plasmid contains a mutant version of the ribosomal protein L3 (RPL3) of Saccharomyces cerevisiae that substantially increases DON resistance when expressed in yeast. Transformants were selected on minimal media lacking leucine and adenine.

The obtained yeast strain was grown to an OD₆₀₀ 0.7 in selective medium (SC-LEU-ADE). Cells were transferred to YPD medium (10% glucose) supplied with additional adenine, grown for 2 h at 30° C, harvested by centrifugation and diluted to an OD₆₀₀ of 3.0 in YPD. DON was added to 5 ml of culture starting with 200 ppm DON. After 3 h the concentration was increased to 400 ppm, after 6 h to 600 ppm, and after 9 h to 1.000 ppm. The cells were incubated for further 15 h at 30°C, before they were harvested, washed three times with ice-cold water, extracted in 2.5 ml of methanol/ water (4:1) and sonicated. After centrifugation the supernatant was filtered through a glass microfibre filter (Whatman 1822 025). 500 µl of the yeast extracts were concentrated to dryness under a constant stream of nitrogen and dissolved in 100 µl HPLC-grade water.

**Heterologous expression of DOGT1 in Escherichia coli.** The DOGT1 protein was expressed in Escherichia coli XL1-blue as a GST fusion. The DOGT1 gene was released from the yeast expression vector by HindIII digestion and Klenow fill in, followed by a NotI digest. The resulting fragment was cloned into the SmaI + NotI sites of the GST gene fusion vector pGEX-4T-3 (Amersham Pharmacia). Recombinant fusion protein was purified using glutathione-coupled Sepharose (Amersham Pharmacia) according to the manufacturer' s instructions.

To test the effect of the N-terminal GST tag on activity, the gene encoding the fusion protein was PCR amplified using DNA polymerase with proof reading activity (Pfu polymerase, MBI) and the fusion protein specific primers GSTDOGpYAK7-fw (5'-TCACCCGG-GAAACAGTAATCATGTCC-3') and GSTDOGpYAK7-rv (5'-CGAGGCAGATCGTCAGT-CAGTC-3'). The PCR product was cloned HindIII+NotI into the yeast expression vector pYAK7. DON-detoxification ability was tested by expression in YZGA515 and application to toxin containing media as described earlier.

**Enzyme assays.** The glucosyltransferase activity assay mix contained 1 µg of recombinant GST-fusion protein, 10 mM 2-mercapto-ethanol, 50 mM Tris/HCl pH 7.0, 0.5 mM radioactive labeled UDP-[¹⁴C] glucose (4.4*10³ cpm, NEN Life Science Products, USA), 0.01% BSA and 1 mM of acceptor substrate (dissolved in DMSO in 20 mM stock solutions). The reactions were carried out in volumes of 20 µl at 30°C for 1 h, stopped by adding 2 µl trichloroacetic acid (240 mg/ml), frozen and stored at -20°C.
Analysis of reaction products was performed by TLC. An aliquot of each sample was spotted on a silica-gel plate (Kieselgel 60; Merck) and developed with a mixture of 1-butanol/1-propanol/ethanol/water (2:3:3:1, v/v/v/v). The intensity of each radioactive spot was determined using a phosphoimager (STORM 860 system, Molecular Dynamics). The plates were additionally stained with p-anisaldehyde (0.5% in methanol /H₂SO₄ /acetic acid, 85:5:10, V/V/V).

**Inhibition of wheat ribosomes in vitro.** To analyze whether the 3-β-D-Glucopyranosyl-4-deoxynivalenol is less phytotoxic than its aglucon, a wheat germ extract coupled in vitro transcription/translation system (TNT Coupled Wheat Germ Extract, T3, Promega) was used. After performing transcription/translation reactions for 25 minutes according to the manufacturers instructions in the presence of either DON, purified DON glucoside (1 µM, 2.5 µM, 5 µM, 10 µM and 20 µM) or water as a control, the activity of the firefly luciferase reporter was determined (Luciferase Assay System, Promega) using a luminometer (Victor 2, Wallac).

**Plant treatment with different stress response related compounds for expression analysis.** For Reverse Transcription (RT)-PCR analysis of mRNA expression of DOGT1 following treatments with DON, salicylic acid (SA), jasmonic acid (JA) and 1-aminocyclopropylcarbonic acid (ACC) seedlings were grown for 2 weeks on vertical MS plates (0.8% phytagar) before they were transferred to liquid MS media. The plants were incubated for 48 h on an orbital shaker (50 rpm) before adding 5 ppm DON, 200 µM SA, 2 µM ACC or 50 µM JA. The compounds were kept in stock solutions dissolved either in 70% ethanol or in DMSO. Treatments with ethanol and DMSO were performed as controls. Plants were harvested at different time points, ground in liquid nitrogen and stored at -70° C until RNA extraction was performed.

**Analysis of mRNA expression of DOGT1 and close homologous by Reverse Transcriptase-PCR.** Total RNA was isolated from plant tissue ground in liquid nitrogen with Trizol Reagent as recommended by the manufacturers (Gibco BRL Life Technologies). RNA was quantified photometrically and visually on a denaturing RNA gel analyzing 5 µg of total RNA.
cDNA was synthesized from 1 µg of total RNA (digested with DNa-seI) with 500 ng of a 18-mer oligo(dT) and the reverse transcriptase SuperScript (Gibco BRL Life Technologies). PCR was performed with approximately 2 µl of the 1:20 diluted cDNA using primers that amplify 200 to 400 bp large fragments located in the C-terminal part of the genes to be analyzed (DOGRT-fw: 5'-ATCCGGGGTTGAACAGCCT-3', DOGRT-rv: 5'-TCAATTATTGGGTTCTGCC-3'; 73C4RT-fw: 5'-GGAGAAAATAGGAGTGTTA-3', 73C4RT-rv: 5'-TCAGTTCTTG-GATTTCACT-3'; 73C6RT-fw: 5'- GAGAAACTGGTCGTACAA-3', 73C6RT-rv: 5'-TCAATTATTGGACTGTGCT-3'; UBQ5-U: 5'-GTCCTTCTTTCTGGTAAACGT-3', UBQ5-D: 5'-AACC CTTGAGGTTGAATCATC-3'). To compare relative amounts of transcripts in the samples, DNA fragments of the UBQ5 were first amplified and normalized sample volumes based on the amount of products corresponding to the UBQ5 transcripts were used for PCR.

**Cloning of plant overexpression constructs and GUS-fusion constructs.** For constitutive overexpression of c-Myc-tagged DOGT1 protein in Arabidopsis, the vector pBP1319 was constructed. It is derived from a modified version of the plant expression vector pPZP221 (25). The promoter cassette consisting of two copies of the 35S-promoter and the polyadenylation signal of CaMV strain Cabb B-D was used from the vector p2RT, a modified version of pRT100 (26).

The c-Myc-tagged DOGT1-fragment was released by SmaI + NotI digestion (Klenow filled) from the yeast expression vector and cloned into pBluescript SKII+, which was cut with ClaI+SmaI and treated with Klenow enzyme. In the next step, the gene was excised from the resulting plasmid as SalI + BamHI fragment and inserted in the XhoI + BamHI sites of p2RT. The obtained 2x35S c-Myc-DOGT1 cassette was isolated by PstI digestion and cloned into the unique PstI site of pPZP221 after the multiple cloning site in that vector had been destroyed by digesting the plasmid with EcoRI+SalI, filling the sites with Klenow and religating it. In the resulting vector pBP1319, the 2x35S c-Myc-DOGT1 cassette is orientated in the opposite direction than the 2x35S gentamycin resistance marker.

For construction of a transcriptional DOGT1-GUS fusion, the GUS vector pPZP-GUS.1 which originates from pPZP200 and contains the GUS gene from pBI101.1 (inserted HindIII + EcoRI into the MCS), was used (27). The DOGT1 promoter region was PCR amplified from genomic DNA using DNA polymerase with proof reading activity (Pfu Polymerase, MBI) and specific primers (DOGP-GUS-fw: 5'-GT-TAAAAGCTTACATGTGCATTACGGTCTGTGTGAATA, DOGP-GUS-rv: 5'-TTTCG-GATCCCATG ATTCAACCTTAGTAAGAAACTCTC). The resulting product was cloned in frame with the GUS gene HindIII + BamHI into the pPZP-GUS.1 vector. Constructs were confirmed by DNA sequencing.

**Generation and analysis of transgenic A. thaliana.** For all plant transformations, the recA deficient Agrobacterium tumefaciens strain UIA143 (28) which harbors the helper plasmid pMP90 (29) was used. A. thaliana was transformed applying the floral dip technique (30). The progeny of 15 independent transformants was selected through three generations to obtain homozygous lines.

For immunodetection of c-Myc-tagged DOGT1, about 200 mg - 500 mg of plant material were homogenized in liquid nitrogen. 300 µl of extraction buffer (200 mM Tris-HCl pH 8.9; 200 mM KCl; 35 mM MgCl₂; 12.5 mM EGTA; 15 mM DTT; 0.6 mM sorbitol) and 15 µl of proteases inhibitor cocktail (Sigma, # 9599) were added to the still frozen samples and the mixture was incubated under vigorous shaking for 15 minutes at 4°C. After centrifugation (14.000 rpm for 15 minutes at 4°C), 200 µl of the supernatants were transferred into fresh tubes and stored at -20°C. Equivalent amounts of protein (50 µg) were used for Western blot analysis which was carried out using primary anti c-Myc antibody purified from hybridoma supernatant (clone 9E10).

For analysis of DON resistance, seeds of homozygous lines exhibiting high DOGT1-expression and Col-0 as control were germinated on MS media containing different concentrations of DON (5 - 30 ppm). Seedlings were grown for 5 weeks before the phenotype was documented. GUS activity was analyzed by staining seedlings or organs of adult plants in X-Gluc solution for 2 to 4 h at 37°C (31).

### RESULTS

**Isolation of the DON-glucosyltransferase (DOGT1) by heterologous expression in yeast.** An unbiased functional screen based on heterologous expression of cDNAs in yeast was set up with the goal to identify plant genes that contribute to resistance against mycotoxins. Wild-type Saccharomyces cerevisiae is highly resistant to deoxynivalenol (DON). In order to reduce the amount of toxin necessary for the screen, a strain deficient in four ABC transporters was generated, which are to a large extent responsible for pleiotropic drug resistance in yeast (32). Strain YZGA452 (snq2Δ::hisG pdr5Δ::TRP1 pdr10Δ::hisG yor1Δ::hisG) is hypersensitive to a wide range of different xenobiotic substances and natural products, including DON. YZGA452 was transformed with a cDNA expression library of A. thaliana (20), where cDNAs are constitutively expressed under the control of the yeast phosphoglucerate kinase promoter. Ten million transformants were generated and diluted pools of transformants were plated on DON containing medium. After selection of DON resistant yeast colonies and confirmation of plasmid dependency of the phenotype, the insert was subcloned and sequenced.

**DOGT1 is a member of the UDP-glucosyltransferase family of A. thaliana and exhibits high similarity to salicylic acid and wound inducible genes of other species.** The cDNA conferring resistance had a size of 1.75 kb and contained an open reading frame of 1488 bp length encoding a putative uridine diphosphate (UDP)-glucosyltransferase. The identified DON-glucosyltransferase (DOGT1) corresponds to gene UGT73C5 and belongs to subfamily 73C, part of group D of UDP-glucosyltransferases (UGTs) of A. thaliana (33). Arabidopsis UGTs constitute a very large gene family, that has been divided into 14 distinct groups, believed to have originated from common ancestors (17). DOGT1 is located in a cluster (Figure 1B) together with five other members of subfamily 73C on chromosome II (BAC clone F13K3, At2g36800). All six tandemly repeated genes contain no introns, and are highly similar to each other (77 - 89% identity at the amino acid level). The similarity is also very high in the intergenic promoter regions.

A database search with the DOGT1 amino acid sequence revealed high similarity to glucosyltransferases from tobacco (TOGT1, ref. 34; Is5a and Is10a, ref. 35) and tomato (Twi-1, ref. 36), the expression of which has been shown to be elevated following treatment with salicylic acid (SA), fungal elicitors or wounding (34, 36, 37), and to the betanidin 5-O-glucosyltransferase of Dorotheanthus bellidiformis (38). Two putative, uncharacterized glucosyltransferases from Vigna angularis (ADGT-9) and Oryza sativa with homology to DOGT1 were also included in the amino acid alignment shown in Figure 2. Regions of high similarity were observed in both amino- and carboxy-terminal domains of the deduced amino acid sequences. Indicated in Figure 2 are the hypothetical acceptor substrate binding region (38) and the UGT consensus sequence (33).

**The expression of DOGT1 is developmentally regulated and induced by DON and other stress response related compounds.** To investigate whether the expression of DOGT1 is regulated in a similar fashion as described for the related genes of other plant species, the ORF of the β-glucuronidase reporter gene was placed behind the DOGT1 promoter (P_{DOGT1}-GUS). The tissue specific expression of the transcriptional GUS fusion was examined histochemically in transgenic Arabidopsis homozygous for the fusion gene. The results shown in Figure 3 demonstrate that DOGT1 expression is regulated developmentally and is overall rather low. In seedlings, GUS activity was observed to be root and hypocotyl specific, with the strongest expression in the vascular system, in meristematic tissue of the root tips (in the primary root as well as in lateral roots) and in the vasculature of the hypocotyl right after germination. Staining in the vasculature decreased significantly later in development and a patchy staining pattern appeared in epidermal root cells. In adult plants GUS activity was detected in late stages of flower development in petals and in abscission zones (Figure 3A).

Exposure of seedlings to either DON (5 ppm for 4 h) or the ethylene precursor aminocyclopropylcarbonic acid (ACC, 2 µM for 1 h) was found to induce P_{DOGT1}-GUS expression (Figure 3). No induction of expression of the reporter was detected upon SA-treatment (200 µM for 12 h) or treatment with jasmonic acid (JA, 50 µM for 1 h). Semiquantitative reverse transcriptase PCR was applied to validate the results obtained from GUS reporter analyses by detecting changes in mRNA levels of DOGT1, 73C4 and 73C6 following treatment with the same concentrations of DON, SA, ACC and JA as used before.

As shown in Figure 3C the results of the reverse transcriptase PCR confirm the DON inducible expression of DOGT1 previously observed with the reporter construct, and furthermore show that also the other two tested UGTs are DON inducible. An increase in the amounts of transcripts was observed already after 1 h of incubation with the toxin, reaching a peak after 4 h and declining again between 6 and 12 h. Interestinglu, 73C6 showed a stronger induction of expression by DON than DOGT1 or 73C4 although the data shown below indicate that it does not accept the toxin as a substrate. After SA treatment, DOGT1, 73C4 and 73C6 expression was evident at low levels at 4 h and slightly stronger at 12 h. It has to be noted that the applied concentration of 200 µM SA induced the expression of the 3 genes rather weakly. Jasmonic acid as well as treatment with ACC also lead to weak induction of expression of DOGT1 and UGT73C6 apparent already after 1 h of treatment, but rapidly declining with no transcript accumulation detectable anymore after 2 h of exposure to the compounds (Figure 3C).

Phenotypic determination of the spectrum of trichothecene resistance in yeast. Trichothecene toxicity in animals depends on the hydroxylation pattern, as well as on the position, number and complexity of esterifications (5). The basic trichothecene structure and numbering system of carbon atoms is shown in Figure 1A. Members of the subclass B (e.g. DON and NIV), contain a keto group at carbon C-8, while type A trichothecenes, (e.g. the highly toxic T-2 toxin produced by F. sporotrichoides) do not. Extremely toxic are also macrocyclic trichothecenes, like veruccarin A, which contain a macrocyclic ring with ester bonds bridging carbon 4 and carbon 15. Yeast pdr5 mutants are hypersensitive to all the trichothecenes tested so far, allowing us to investigate the ability of DOGT1 and other genes in the cluster to confer resistance to various members of the trichothecenes.

The high similarity of UGT73C1, C2, C3, C4, C5 (DOGT1) and C6 and their clustered appearance on the chromosome suggests that they have evolved via gene duplication from one ancestral gene and might therefore have related enzymatic properties. To analyze possible similarities in their function, the six ORFs were amplified with specific primers and expressed in yeast as fusion proteins with an N-terminal c-Myc epitope tag. Comparison of transformants expressing tagged or untagged DOGT1 showed that the epitope did not interfere with the DON-protective activity. The yeast transformants representing the full gene set of the cluster were spotted on media containing increasing concentrations of various trichothecenes. Transformants containing the empty expression vector were used as controls.

The results shown in Figure 1 revealed that UGT73C4, like DOGT1, confers DON resistance. Although C4 has only the second highest similarity (79% identity to DOGT1), it had the ability to detoxify DON and 15-ADON (Figure 1C), but failed to confer resistance to 3-ADON, NIV, TTC, HT-2-toxin, T2-toxin, DAS and VA. In contrast, UGT73C6 which exhibits with 89% identity on amino acid level the highest similarity did not protect against any of the tested trichothecenes. Likewise, 73C3 was not able to confer resistance to the tested mycotoxins although all of the four proteins were expressed to a similar extent in yeast. The 73C1 and 73C2 constructs did also not enhance toxin resistance, however this could also be due to lower recombinant protein levels present in the yeast transformants (Figure 1D).

**DON detoxification specificity is located in the N-terminal part of DOGT1.** The different characteristics of the closely related enzymes despite their high sequence similarity opened the possibility to elucidate features of the proteins which are crucial for DON recognition. An EcoRI site present in both DOGT1 and 73C6 (see Figure 4A) was used to construct hybrids containing the N-terminal part of one and the C-terminal part of the respective other gene. Expression in yeast and exposure to DON containing media revealed that the N-terminus of DOGT1 (consisting of the first 189 aa) is essential for the ability to detoxify DON (Figure 4B).

A lysine residue at position 136 in DOGT1 is conserved in the detoxifier 73C4 but replaced by other amino acids in the two nondetoxifying homologues C3 and C6. Site directed mutagenesis was used to introduce mutations into DOGT1 and the UGT73C6 leading to a change of the lysine residue 136 in DOGT1 to glutamic acid, which is present at the corresponding position in C6 (amino acid 137). The mutant DOGT1^{K136E} protein protected with the same efficiency as DOGT1, demonstrating that K136 is not essential for the DON detoxification ability. Also the reverse construct 73C6^{E137K} remained inactive.

**In vivo and in vitro analysis proof that DOGT1 catalyses the transfer of glucose from UDP-glucose specifically to the 3-OH position of DON.** The protection against 15-ADON and the inability to confer resistance against 3-ADON suggested that DOGT1 may catalyse the formation of DON-3-O-glucoside. To test this hypothesis, first chemically synthesized DON derivatives with the glucose moiety was attached either to the C3- or C15-hydroxyl group of DON. The two products were characterized with LC-MS/MS.

The DON-3-O-glucoside and DON-15-O-glucoside eluted at 12.43 min and 12.68 min, respectively. The mass spectra of the glucosides showed characteristic differences in their fragmentation pattern (Figure 5). While the DON-3-O-glucoside fragmented to an ion of 427.2 m/z under the given conditions, the same ion was not detected with DON-15-O-glucoside. The loss of 30 atomic mass units can be explained by the cleavage of the -CH₂OH group at C₆, which is prevented when the hydroxyl group is conjugated with glucose as in DON-15-O-glucoside. Further breakdown (MS) of the DON-glucosides in the linear ion trap showed an almost identical fragmentation pattern as that of the [DON-H]- ion (295.3 m/z, not fragmented in Q2), confirming the presence of DON entities in the reaction products.

With these tools at hand it was possible to directly determine which glucoside was formed in yeast cells. Yeast strain YZGA515, incapable of converting DON into 3-ADON due to a deletion of the yeast acetlytransferase gene AYT1, was transformed with both the DOGT1 expression vector and a plasmid containing a gene encoding a trichothecene insensitive mutant ribosomal protein L3, the target of trichothecenes, to increase DON tolerance of yeast cells. After the resulting strain had been incubated with high amounts of DON, reaching a final concentration of 1000 ppm in the medium, the DON-metabolite was extracted from the cells and identified as the expected 3-O-glucopyranosyl-4-deoxynivalenol (Figure 6A) by HPLC and mass spectroscopy. Figure 5 shows the fragmentation pattern of the synthesized reference substances and the product peak from yeast expressing DOGT1. As expected, the metabolite was not present in the control strain, lacking DOGT1 activity.

In order to further verify substrate specificity, a GST-DOGT1 fusion was constructed. The GST-DOGT1 fusion gene was also expressed in yeast, where it conferred DON resistance like wild-type DOGT1. The gene product was expressed in E. coli and affinity purified. The reaction products generated in vitro during incubation of either the DOGT1 fusion-protein or GST with UDP-[¹⁴C] glucose and DON were analyzed using TLC. A spot with the same Rf value as the synthesized DON-glucoside was observed in the reaction containing the GST-DOGT1 fusion protein but not in the control.

**Glucosylation of 4-deoxynivalenol severely reduces its toxicity.** To analyze whether the 3-β-D-Glucopyranosyl-4-deoxynivalenol is less phytotoxic than DON, a wheat germ extract coupled in vitro transcription/translation system was used. As shown in Figure 6B the presence of 1 µM DON in the reaction inhibited protein translation significantly, leading to 36.8% reporter enzyme activity compared to the control (100%). 5 µM of the toxin resulted in only 3.1% luminescence remaining whereas 20 µM of the synthesized DON-3-glucoside only inhibited luciferase activity by 8%. These results proof that glucosylation of DON is a detoxification process.

**Overexpression of DOGT1 in Arabidopsis thaliana increases DON resistance.** Transgenic A. thaliana constitutively expressing DOGT1 under the control of a tandem 35S promoter were generated and the amount of recombinant protein in transformants determined by Western blotting utilizing the N-terminal c-Myc epitope-tag. Seeds of the homozygous line 1319/2, which was found to have a high DOGT1 expression level (Figure 6C), and wild-type Col-0 as control were germinated on MS media containing 5 - 30 ppm DON and grown for 5 weeks before the phenotype was analyzed.

The main phytotoxic effect observed was slow germination of wild-type plants. Root formation did not occur at all, cotyledons developed but started to bleach before true leaves could be formed. After 3 weeks of exposure to DON most of the wild-type seedlings had lost all green color and ceased growth. DOGT1-overexpression lines showed also a clear delay in development. Compared to wild-type, germination occurred earlier, roots were formed, cotyledons did not bleach and true leaves appeared. The differences in DON sensitivity observed were most apparent on media containing 15 ppm of toxin (Figure 6D). Control transformants with empty vector (containing solely the gentamycin resistance gene), and lines expressing low amounts of DOGT1 were as sensitive as wild-type Col-0.

### DISCUSSION

**Biotechnological relevance.** Fusarium diseases of wheat and barley are of high economic significance for countries around the world. The United States for instance have been severely hit by Fusarium epidemics in the last decade. Direct losses due to FHB for the US wheat producers have been estimated to average about $260 million annually and over the period 1998-2000 the combined economic losses for small grain cereals were estimated at $2.7 billion (39). Deoxynivalenol contamination of large portions of the harvest can lead to high intake of the mycotoxin. Children are the population group most at risk to exceed the tolerable daily intake level (TDI) for DON. In the problematic year 1998 80% of 1 year old children in the Netherlands exceeded the TDI (7).

The high importance of Fusarium diseases justifies research on mycotoxin inactivation, although resistance against the pathogen is most likely a polygenic trait in crop plants and toxin resistance just one of its components. The production of trichothecenes is the only virulence factor of the fungal pathogen which is experimentally confirmed so far, with the exception of pleiotropic mutations in MAP kinases affecting not exclusively virulence but also conidiation, perithecia formation, vegetative growth and mycotoxin production (40, 41). The present finding that overexpression of DOGT1 leads to increased deoxynivalenol resistance in transgenic Arabidopsis opens new possibilities for biotechnological approaches aiming to antagonize the fungal virulence factor. The only result published so far relies on a Fusarium acetyltransferase (42) converting DON into 3-ADON, which is about 2-fold less toxic to laboratory animals and therefore no detoxification within the meaning of the present invention. The phenotype of transformed yeast and Arabidopsis, as well as data obtained using in vitro translation assays, demonstrate that the DON-glucoside exhibits reduced toxicity. In general, glucosylation converts reactive and toxic aglucones into stable and non-reactive storage forms, thereby limiting their interaction with other cellular components. The addition of a sugar blocks the reactive site of the substance and consequently reduces toxicity for the plant. Such modifications are furthermore considered to provide access to membrane-bound transporters opening exit pathways from the cytosol to for example the cell wall or the vacuole (43).

Glucosylation of trichothecenes represents a detoxification process for plants. Yet, in the digestive tract of humans and animals the mycotoxin-glucoconjugates could be easily hydrolyzed, regenerating the toxin. The extent to which the DON-glucoside is transported to the vacuole or to the apoplast is currently unknown. Before one attempts to use transgenic plants overexpressing a DON-glucosyltransferase, it should be investigated whether only the vacuolar glucoside or also toxin bound to cell wall material as "insoluble residue" is a source of "masked mycotoxin" (44), which escapes traditional analytical techniques and may be of much higher toxicological relevance as currently assumed. The analytical tools and reference materials developed during this work is suitable to address these questions.

Inactivation of toxins by glucosylation seems to be a prominent natural mechanism for resistance, enabling plants to cope with the enormous diversity of toxic microbial metabolites they may encounter in nature. DOGT1 is one of 118 UGT genes in A. thaliana which are predicted to conjugate small molecules.

The expression of UGT genes in yeast is a valuable complementary approach to the widely used expression in E. coli, especially when the respective chemicals have targets in eukaryotes only. One problem is that wild-type yeast cells are frequently "impermeable" to the substances of interest. Inactivating several ABC-transporters in the present host strain was a prerequisite for the approach to select resistance conferring cDNAs. In the case of trichothecenes this allowed the phenotypic detection of the activity of the expressed genes in a simple plate assay. In principle, this approach could be adopted for many other substances.

**Gene evolution and substrate specificity.** DOGT1 is located in a cluster (Figure 1B) with 5 other members of family 73C on chromosome II (At2g36800) indicative for gene duplication by unequal recombination events (32). One could speculate that such a gene amplification event provides a selective advantage under toxin stress. DOGT1 and other members in the cluster have very similar protein sequences suggesting redundancy in enzymatic function. However, when testing the homologues for detoxification of trichothecenes, it was found that yeast expressing the gene with the highest sequence similarity UGT73C6 was as sensitive as wild-type to all toxins tested (as well as UGT73C3), while the second closest homologue UGT73C4 exhibited the same properties as DOGT1. Enzymatic activity towards different hydroxycoumarins has been shown for all 73C cluster members (45), indicating that the UGTs lacking DON-protective activity are not simply loss of function alleles. The cluster is therefore an example that supports the hypothesis that duplicated UGT genes can acquire new substrate specificities and functions during evolution.

Plant UDP-glucosyltransferases are structurally very similar in their carboxy-terminal signature motif to mammalian UGTs which use UDP-glucuronic acid instead of UDP-glucose as donor substrate. The mammalian enzymes play a central role in metabolism and detoxification of chemicals like carcinogens or hydrophobic drugs. Higher plant UGTs have been found to be involved in a parallel range of activities also modifying xenobiotic substances such as herbicides and other pesticides (46, 47). [One open question is whether these detoxification reactions are side-activities of UGTs conjugating endogenous plant compounds. Although recent publications are in favor of the hypothesis that UGTs responsible for the conversion of endogenous substrates may also account for the capacity of plants to detoxify xenobiotic substances (48) this question will need further work to be solved.]

The finding that DOGT1 protects against DON but not NIV, which possesses one additional hydroxyl group at residue 4 (Figure 1A), indicates that the variation of the hydroxylation pattern of the trichothecene backbone might be an important mechanism of the fungus to escape such detoxification reactions. It is unknown whether plants can also conjugate NIV, which has higher toxicity than DON in animal systems (5). The lack of activity against NIV stronglu argues against use of a single DOGT1-like gene in transgenic plants, as NIV producing chemotypes of F. graminearum could get a selective advantage.

The construction of hybrid genes by shuffling the N- and C-terminal part of DOGT1 and UGT73C6 and their expression in yeast proved that the N-terminal 189 amino acids of DOGT1 are essential for its ability to detoxify DON, in agreement with the hypothesis that substrate binding may occur in a conserved hydrophobic area between amino acid 130 and 150 (38). Comparison of two genes with protective effect and two genes without activity, but similar expression level, suggested that a lysine, which is present at amino acid position 136 in DOGT1 (and at the corresponding position in UGT73C4) but replaced in the two UGT's unable to detoxify trichothecenes, could be important for DON detoxification. This hypothesis was falsified using site directed mutagenesis and expression of the engineered proteins in yeast.

Information on specific domains or amino acids involved in acceptor binding of DOGT1 may be potentially useful to search for glucosyltransferases capable to detoxify DON in crop plants like wheat or maize. It should be considered though that attempts to assign functions to UGTs based exclusively on amino acid similarity are highly error prone, as shown in the case of the 73C cluster. Nevertheless, the functionality of candidate genes can be easily tested by expression in yeast. One possibility which can not be excluded is that glucosides of trichothecenes are formed in crop plants by structurally fairly dissimilar UGTs. For instance, UGT84B1 was shown to exhibit the highest in vitro activity against the auxin indole-3-acetic acid (IAA) of all A. thaliana UGTs tested, yet this enzyme does not display the highest sequence similarity to the functional homologue in maize, the iaglu gene (49).

**Regulation of gene expression.** Genes with high sequence similarities to DOGT1 from tobacco and tomato have been shown to be induced by salicylic acid or wounding (34, 36, 37). The analysis of expression of DOGT1 and 73C6 following SA and JA treatment showed that they responded weekly with elevated mRNA levels to SA, JA and the ethylene precursor ACC. Inducibility of gene expression by SA, JA or ethylene is considered to be indicative for a possible role of the upregulated gene product in plant stress or defense responses (50).

Using analysis of mRNA levels and a GUS-reporter construct it was possble to show that DOGT1 transcription in wild-type A. thaliana is developmentally regulated and is rapidly and significantly induced in response to DON exposure. Induction of expression by the mycotoxin has also been observed for other 73C cluster members, regardless of their DON-protective activity. It would be interesting to clarify whether the inducibility is compound specific or a general phenomenon for protein biosynthesis inhibitors. The "ribotoxic stress response" is currently actively investigated by researchers in human cells (51), where the mycotoxin induces the expression of cyclooxigenase-2, a key enzyme in the synthesis of mediators of the inflammatory response, via MAP kinase mediated signaling. The DON inducible expression of DOGT1 is an attractive starting point for investigating whether similar mechanisms exist in plants.

With the present invention it was shown that the huge gene family of glucosyltransferases, especially - as shown in the examples - UGTs plays an important role in plant pathogen interaction by participating in detoxification of metabolites produced by microbes to increase their virulence on hosts. Similar to the large family of leucine-rich-repeat type resistance genes (52) involved in pathogen recognition, gene amplification and diversifying selection of UGTs could lead to a broad spectrum protection against fungal toxins. The selective pressure to escape such glucosylation reactions may be a driving force in evolution of microbial biosynthetic reactions leading to a wide spectrum of toxin structures, as observed for trichothecenes.

### REFERENCES

1. McMullen, M.P., Jones, R. and Gallenberg, D. (1997) Plant Dis. 81, 1340-1348
2. Grove, J. F. (1996) Progress in the Chemistry of Organic Natural Products, pp. 1-70, Springer, Wien
3. Cundliffe, E. and Davies, J.E. (1977) Agents Chemother. 11, 491-499
4. O'Donnel, K., Kistler, H.C., Tacke, B.K. and Casper, H.H. (2000) PNAS 97, 7905-7910
5. Betina, V. (1989) Mycotoxins - chemical, biological and environmental aspects. pp. 192-241, Elsevier, Amsterdam
6. Canady, R.A., Coker, R.D., Egan, K., Krska, R., Kuiper-Goodman, T., Olsen M., Pestka, J., Resnik, S., & Schlatter, J. (2001) WHO Food Additives Series 47, 419-528
7. Codex Committee on Food Additives and Contaminants (2002) Joint FAO/WHO Food Standards Programme, CX/FAC 03/35, www.codex-alimentarius.net
8. McCormick, S.P. (2003) Fusarium head blight of wheat, pp. 165-183, American Phytopathological Society, St. Paul, Minnesota, USA
9. Desjardins, A.E., Procter, R.H., Bai, G., McCormick, S.P., Shaner, G., Buechley, G. and Hohn, T. (1996) Mol. Plant-Microbe Interact. 9, 775-781
10. Bai, G.H, Desjardins, A.E. and Plattner R.D. (2002) Mycopathologia 153, 91-98
11. Kang, Z. and Buchenauer, H. (1999) Physiol. Molec. Plant Pathol. 55, 275-288
12. Mesterhazy, A. (2003) Fusarium head blight of wheat, pp. 363-380, American Phytopathological Society, St. Paul, Minnesota, USA
13. Coleman, J.O.D., Blake-Kalff, M.M.A. and Davies, T.G.E. (1997) Trends Plant Sci. 2, 144-151
14. Scott, P.M., Nelson, K., Kanhere, S.R., Karpinski, K.F., Hayward, S., Neish, G.A. and Teich, A. (1984) Appl. Environ. Microbiol. 48, 884-886
15. Miller, J.D. and Arnison, P.G. (1986) Can J Plant Path 8, 147-150
16. Sewald, N., von Gleissenthall, J. L., Schuster, M., Müller, G. and Aplin, R.T. (1992) Tetrahedron Asymmetry 3, 953-960
17. Ross, J., Yi, L., Lim, E.-K. and Bowles, D. (2001) Genome Biol. 2, 3004.1-3004.6.
18. Sikorski, R.S. and Hieter, P. (1989) Genetics 122, 19-27
19. Murashige, T. and Skoog, F. (1962) Plant Physiol. 15, 473-497
20. Minet, M., Dufour, M.-E. and Lacroute, F. (1992) Plant J. 2, 417-422
21. Ballester, R., Michaeli, T., Ferguson, K., Xu, H.-P., McCormick F, and Wigler, M. (1989), Cell 59, 681-686
22. Egner, R., Mahe, Y., Pandjaitan, R. and Kuchler, K. (1995) Mol Cell Biol 15, 5879-5887
23. Pogulis, R.J., Vallejo, A.N. and Pease, L.R. (1996) Methods Mol Biol 57, 167-176
24. Savard, M. (1991) J. Agr. Food Chem., 39, 570-574
25. Hajdukiewicz, P., Svab, Z. and Maliga, P. (1994) Plant Mol Biol 25, 989-994
26. Töpfer, R., Matzeit, V., Groneborn, B., Schell, J. and Steinbiss H.-H. (1987) Nucleic Acid Research 14, 5890
27. Diener, A.C., Li, H., Zhou, W., Whoriskey, W.J., Nes, W.D. and Fink, G.R. (2000) Plant Cell 12, 853-870
28. Farrand, S.K., O'Morchoe, S.P. and McCutchan, J. (1989) J Bacteriol 171, 5314-5321
29. Koncz, C. and Schell, J. (1986) Mol Gen Genet 204, 383-396
30. Clough, S.J. and Bent, A.F. (1998) Plant J. 16, 735-743
31. Jefferson, R.A. (1987) Plant Mol. Biol. Rep. 5, 387-405
32. Wolfger, H., Mamnun, Y.M. and Kuchler, K. (2001) Res Microbiol. 152, 375-89
33. Li, Y., Baldauf, S., Lim, E.-K., and Bowles, D.J. (2001) J. Biol Chem 276, 4338-4343
34. Fraissinet-Tachet, L., Baltz, R., Chong, J., Kauffmann, S., Fritig, B. and Saindrenan, P. (1998) FEBS 437, 319-323
35. Horvath, D.M. and Chua, N.-H. (1996) Plant Mol Biol 31, 1061-1072
36. Truesdale, M.R., Doherty, H.M., Loake, G.J., McPherson, M.J., Roberts, M.R. and Bowles, D.J. (1996) Plant Physiol 112, 446
37. Horvath, D., Huang, D.J. and Chua, N.-H. (1998) Mol. Plant-Microbe Interact. 11, 895-905
38. Vogt, T., Grimm, R. and Strack, D. (1999) Plant J 19, 509-519
39. Nganje, W.E., Johnson, D.D., Wilson, W.W., Leistritz, F.L., Bangsund, D.A. and Tiapo, N.M. (2001) Agribusiness and Applied Economics Report 464
40. Jenczmionka, N.J., Maier, F.J., Losch, A.P. and Schafer, W. (2003) Curr. Genet. 43, 87-95
41. Hou, Z., Xue, C., Peng, Y., Katan, T., Kistler, H.C. and Xu, J.R. (2002) Mol Plant Microbe Interact. 15, 1119-1127
42. Okubara, P.A., Blechl, A.E., McCormick, S.P., Alexander, N.J., Dill-Macky, R. and Hohn, T.M. (2002) Theor Appl Genet. 106, 74-83
43. Jones, P. and Vogt, T. (2001) Planta 213, 164-174
44. Engelhardt, G., Ruhland, M. and Wallnöfer, P.R. (1999) Adv. Food Sci. 21, 71-78
45. Lim, E.-K., Baldauf, S., Li, Y., Elias, L., Worrall, D., Spencer, S.P., Jackson, R.G., Taguchi, G., Ross, J. and Bowles, D. (2003) glucobiol. 13, 139-145
46. Wetzel, A. and Sandermann, H. (1994) Arch. Biochem. Biophys. 314, 323-328
47. Leah, J.M., Worral, T.L. and Cobb, A.H. (1992) Pest. Science 34, 81-87
48. Meßner, B., Thulke, O. and Schäffner, A.R. (2003) Planta 217, 138-146
49. Jackson, R.G., Lim, E.-K., Li, Y., Kowalczyk, M., Sandberg, G., Hoggett, J., Ashford, D.A. and Bowles, D. (2001) J. Biol. Chem. 276, 4350-4356
50. Reymond, P. and Farmer, E.E. (1998) Curr Opin Plant Biol. 1, 404-411
51. Laskin, J.D., Heck, D.E. and Laskin, D.L. (2002) Toxicol Sci. 69, 289-291
52. Meyers, B.C., Kozik, A., Griego, A., Kuang, H. and Michelmore, R.W. (2003) Plant Cell 15, 809-834
53. Eudes F., Comeau, A., Rioux S. and Collin J. (2000) Can. J. Plant Path. 22, 286-292
54. Potrykus, I. and Spangenberg, G. (Eds.): Gene Transfer to Plants. Springer Verlag Berlin (1995), ISBN 3-54058406-4

### Abbreviations:

FHB, Fusarium head blight; DON, deoxynivalenol; 15-ADON, 15-acetyl-deoxynivalenol; NIV, nivalenol; UGT, UDP-glucosyltransferase; ABC, ATP-binding cassette; DOGT1, DON-glucosyltransferase; ORF, open reading frame; GUS, β-glucuronidase; SA, salicylic acid; JA, jasmonic acid; ACC, 1-aminocyclopropylcarbonic acid; TDI, tolerable daily intake; IAA, indole-3-acetic acid; DAG, days after germination.

## Claims

1. Method for detoxification of mycotoxins **characterised in that** a mycotoxin is contacted with a glucosyltransferase in the presence of an activated glucose.

2. Method according to claim 1, **characterised in that** said glucosyltransferase is UDP-glucosyltransferase and that said activated glucose is UDP-glucose.

3. Method according to claim 1 or 2, **characterised in that** the mycotoxin is selected from trichothecenes, especially trichothecenes having a free hydroxy-group at position C3 and two hydrogen groups at C4.

4. Method according to any one of claims 1 to 3, **characterised in that** said mycotoxin is deoxynivalenol, 15-acetyl-deoxynivalenol, 15-Acetoxyscirpendiol or mixtures thereof.

5. Method according to any one of claims 1 to 4, **characterised in that** it is performed in vivo using recombinant DNA technology and/or glucosyltransferase expression enhancing compounds.

6. Method according to claim 5, **characterised in that** said glucosyltransferase is expressed in a transgenic plant cell containing a recombinant glucosyltransferase and/or a recombinant regulating region for a glucosyltransferase.

7. Method according to claim 5 or 6, **characterised in that** a mycotoxin-inducible promoter of a glucosyltransferase is used.

8. Method according to any one of claims 1 to 4, **characterised in that** said glucosyltransferase is immobilised to a solid surface and a mycotoxin containing solution or suspension is contacted with said immobilised glucosyltransferase.

9. Method according to any one of claims 1 to 8, **characterised in that** said glucosyltransferase is an UDP-glucosyltransferase corresponding to subfamily 73C of Arabidopsis thaliana, especially UDP-glucosyltransferases 73C5 and 73C4.

10. A recombinant cell being resistant to mycotoxins, especially to trichothecenes, comprising a heterologous glucosyltransferase or an enhanced expression activity of an endogeneous glucosyltransferase due to transgenic expression regulating elements and at least one, preferably three or more, ABC transporters which are reduced or inactivated in their activity, especially pdr5 deletions.

11. A cell according to claim 10 **characterised in that** it is a plant cell or a yeast cell.

12. A cell according to claim 10 or 11 **characterised in that** it has a deletion in pdr5, pdr10, snq2, yor1, pdr15, ayt1 or combinations of these deletions.

13. Use of a cell according to any one of claims 10 to 12 in a method according to any one of claims 1 to 9.

14. Use according to claim 13, **characterised in that** said plant or plant cell is resistant to Fusarium.

15. Use of a cell according to any one of claims 10 to 12 for detoxification of mycotoxins in mycotoxin containing solutions or suspensions, especially for detoxification of trichothecenes in agriculture or beer production.

16. Use of a cell according to any one of claims 10 to 12 for producing glucosylated mycotoxins, especially glucosylated DON or 15-ADON.

17. Method for producing glucosylated mycotoxins **characterised in that** a mycotoxin is contacted in vivo or in vitro with a glucosyltransferase in the presence of an activated glucose.

18. Method according to claim 17 **characterised in that** a step according to any one of claims 2 to 9 is performed.

## Patentansprüche

1. Verfahren zur Entgiftung von Mykotoxinen, **dadurch gekennzeichnet, dass** ein Mykotoxin mit einer Glucosyltransferase in Gegenwart einer aktivierten Glucose in Kontakt gebracht wird.

2. Verfahren nach Anspruch 1, **dadurch gekennzeichnet, dass** die Glucosyltransferase UDP-Glucosyltransferase ist und dass die aktivierte Glucose UDP-Glucose ist.

3. Verfahren nach Anspruch 1 oder 2, **dadurch gekennzeichnet, dass** das Mykotoxin ausgewählt ist aus Trichothecenen, insbesondere Trichothecenen mit einer freien Hydroxy-Gruppe an Position C3 und zwei Wasserstoff-Gruppen an C4.

4. Verfahren nach einem der Ansprüche 1 bis 3, **dadurch gekennzeichnet, dass** das Mykotoxin Desoxynivalenol, 15-Acetyl-desoxynivalenol, 15-Acetoxyscirpendiol oder Mischungen davon ist (sind).

5. Verfahren nach einem der Ansprüche 1 bis 4, **dadurch gekennzeichnet, dass** es *in vivo* unter Verwendung rekombinanter DNA-Technologie und/oder Glucosyltransferase-Expression verstärkenden Verbindungen durchgeführt wird.

6. Verfahren nach Anspruch 5, **dadurch gekennzeichnet, dass** die Glucosyltransferase in einer transgenen Pflanzenzelle exprimiert wird, die eine rekombinante Glucosyltransferase und/oder einen rekombinanten Regulierungsbereich für eine Glucosyltransferase enthält.

7. Verfahren nach Anspruch 5 oder 6, **dadurch gekennzeichnet, dass** ein Mykotoxin-induzierbarer Promotor einer Glucosyltransferase verwendet wird.

8. Verfahren nach einem der Ansprüche 1 bis 4, **dadurch gekennzeichnet, dass** die Glucosyltransferase an einer festen Oberfläche immobilisiert wird und eine Mykotoxin enthaltende Lösung oder Suspension mit der immobilisierten Glucosyltransferase in Kontakt gebracht wird.

9. Verfahren nach einem der Ansprüche 1 bis 8, **dadurch gekennzeichnet, dass** die Glucosyltransferase eine UDP-Glucosyltransferase ist, die der Unterfamilie 73C von Arabidopsis thaliana entspricht, insbesondere UDP-Glucosyltransferasen 73C5 und 73C4.

10. Rekombinante Zelle, die gegen Mykotoxine, insbesondere gegen Trichothecene, resistent ist, umfassend eine heterologe Glucosyltransferase oder eine verstärkte Expressionsaktivität einer endogenen Glucosyltransferase aufgrund von eine transgene Expression regulierenden Elementen und mindestens einen, vorzugsweise drei oder mehr, ABC-Transportern, die in ihrer Aktivität reduziert oder inaktiviert sind, insbesondere durch pdr5-Deletionen.

11. Zelle nach Anspruch 10, **dadurch gekennzeichnet, dass** sie eine Pflanzenzelle oder eine Hefezelle ist.

12. Zelle nach Anspruch 10 oder 11, **dadurch gekennzeichnet, dass** sie eine Deletion in pdr5, pdr10, snq2, yor1, pdr15, ayt1 oder Kombinationen dieser Deletionen aufweist.

13. Verwendung einer Zelle nach einem der Ansprüche 10 bis 12 bei einem Verfahren nach einem der Ansprüche 1 bis 9.

14. Verwendung nach Anspruch 13, **dadurch gekennzeichnet, dass** die Pflanze oder Pflanzenzelle gegen Fusarium resistent ist.

15. Verwendung einer Zelle nach einem der Ansprüche 10 bis 12 zur Entgiftung von Mykotoxinen in Mykotoxin-hältigen Lösungen oder Suspensionen, insbesondere zur Entgiftung von Trichothecenen in der Landwirtschaft oder in der Biererzeugung.

16. Verwendung einer Zelle nach einem der Ansprüche 10 bis 12 zur Herstellung glucosylierter Mykotoxine, insbesondere von glucosyliertem DON oder 15-ADON.

17. Verfahren zur Herstellung von glucosylierten Mykotoxinen, **dadurch gekennzeichnet, dass** ein Mykotoxin *in vivo* oder *in vitro* mit einer Glucosyltransferase in Gegenwart einer aktivierten Glucose in Kontakt gebracht wird.

18. Verfahren nach Anspruch 17, **dadurch gekennzeichnet, dass** ein Schritt nach einem der Ansprüche 2 bis 9 durchgeführt wird.

## Revendications

1. Procédé la détoxification de mycotoxines, **caractérisé en ce qu'**une mycotoxine est mise en contact avec une glucosyltransférase en présence d'un glucose activé.

2. Procédé suivant la revendication 1, **caractérisé en ce que** ladite glucosyltransférase est l'UDP-glucosyltransférase et **en ce que** ledit glucose activé est l'UDP-glucose.

3. Procédé suivant la revendication 1 ou 2, **caractérisé en ce que** la mycotoxine est choisie parmi des trichothécènes, en particulier des trichothécènes ayant un groupe hydroxy libre en position C3 et deux groupes hydrogène en position C4.

4. Procédé suivant l'une quelconque des revendications 1 à 3, **caractérisé en ce que** ladite mycotoxine est le désoxynivalénol, le 15-acétyl-désoxynivalénol, le 15-acétoxy-scirpendiol ou leurs mélanges.

5. Procédé suivant l'une quelconque des revendications 1 à 4, **caractérisé en ce qu'**il est mis en oeuvre in vivo en utilisant la technologie de l'ADN recombinant et/ou des composés amplifiant l'expression de la glucosyltransférase.

6. Procédé suivant la revendication 5, **caractérisé en ce que** ladite glucosyltransférase est exprimée dans une cellule végétale transgénique contenant une glucosyltransférase recombinante et/ou une région régulatrice recombinante pour une glucosyltransférase.

7. Procédé suivant la revendication 5 ou 6, **caractérisé en ce qu'**un promoteur, inductible par une mycotoxine, d'une glucosyltransférase est utilisé.

8. Procédé suivant l'une quelconque des revendications 1 à 4, **caractérisé en ce** ladite glucosyltransférase est immobilisée à une surface solide et une solution ou suspension contenant une mycotoxine est mise en contact avec ladite glucosyltransférase immobilisée.

9. Procédé suivant l'une quelconque des revendications 1 à 8, **caractérisé en ce** ladite glucosyltransférase est une UDP-glucosyltransférase correspondant à la sous-famille 73C d'Arabidopsis thaliana, en particulier les UDP-glucosyl-transférases 73C5 et 73C4.

10. Cellule recombinante qui est résistante aux mycotoxines, en particulier aux trichothécènes, comprenant une glucosyltransférase hétérologue ou une activité d'expression amplifiée d'une glucosyltransférase endogène en raison de l'expression transgénique d'éléments régulateurs et d'au moins un, de préférence de trois ou plus, transporteurs ABC, dont l'activité est réduite ou inactivée, notamment des délétions pdr5.

11. Cellule suivant la revendication 10, **caractérisée en ce qu'**elle consiste en une cellule végétale ou une cellule de levure.

12. Cellule suivant la revendication 10 ou 11, **caractérisée en ce qu'**elle comporte une délétion dans pdr5, pdr10, snq2, yor1, pdr15, ayt1 ou des combinaisons de ces délétions.

13. Utilisation d'une cellule suivant l'une quelconque des revendications 10 à 12, dans un procédé suivant l'une quelconque des revendications 1 à 9.

14. Utilisation suivant la revendication 13, **caractérisée en ce que** ladite plante ou cellule végétale est résistante au Fusarium.

15. Utilisation d'une cellule suivant l'une quelconque des revendications 10 à 12, pour la détoxification de mycotoxines dans des solutions ou suspensions contenant des mycotoxines, en particulier pour la détoxification des trichothécènes en agriculture ou dans la production de la bière.

16. Utilisation d'une cellule suivant l'une quelconque des revendications 10 à 12, pour la production de mycotoxines glucosylées, notamment de DON ou 15-ADON glucosylée.

17. Procédé pour la production de mycotoxines glucosylées **caractérisé en ce qu'**une mycotoxine est mise en contact in vivo ou in vitro avec une glucosyltransférase en présence d'un glucose activé.

18. Procédé suivant la revendication 17, **caractérisé en ce qu'**une étape suivant l'une quelconque des revendications 2 à 9 est mise en oeuvre.
